(19) Europäisches Patentamt
European Patent Office
Office européen des brevets

(11) **EP 3 950 707 A1**

(12) **EUROPEAN PATENT APPLICATION**
published in accordance with Art. 153(4) EPC

(43) Date of publication:
**09.02.2022 Bulletin 2022/06**

(21) Application number: 20763321.5

(22) Date of filing: 27.02.2020

(51) International Patent Classification (IPC):
**C07K 14/435** (2006.01)       **C07K 14/765** (2006.01)
**A61K 9/00** (2006.01)          **A61K 38/17** (2006.01)
**A61K 47/64** (2017.01)         **A61P 21/00** (2006.01)

(52) Cooperative Patent Classification (CPC):
**A61K 9/00; A61K 38/17; A61K 47/64; A61P 21/00;**
**C07K 14/435; C07K 14/765**

(86) International application number:
**PCT/KR2020/002809**

(87) International publication number:
**WO 2020/175931 (03.09.2020 Gazette 2020/36)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **27.02.2019  KR 20190023375**

(71) Applicant: **Daewoong Pharmaceutical Co., Ltd.**
**Hwaseong-si**
**Gyenoggi-do 18623 (KR)**

(72) Inventors:
• **KOH, Jung Min**
**Seoul 06291 (KR)**
• **KIM, Sung Sub**
**Daejeon 34049 (KR)**
• **AHN, Kyong Hoon**
**Seoul 06978 (KR)**

(74) Representative: **Treeby, Philip David William et al**
**Maucher Jenkins**
**Seventh Floor Offices**
**Artillery House**
**11-19 Artillery Row**
**London SW1P 1RT (GB)**

(54) **COMPOSITION COMPRISING ALBUMIN-COUPLED SLIT3 LRRD2 FOR PREVENTION OR TREATMENT OF MUSCLE DISEASE**

(57)     The present invention relates to a composition comprising albumin-coupled Slit3 LRRD2 for prevention or treatment of muscle disease and more particularly provides a fusion protein comprising albumin-coupled Slit3 LRRD2, a nucleic acid molecule coding for the fusion protein, a recombinant vector carrying the nucleic acid molecule, a transformant anchoring the recombinant vector, a method for preparing a fusion protein by using the transformant, a composition comprising the fusion protein for prevention or treatment of muscle disease, and a composition comprising the fusion protein for improving in vivo half-life of LRRD2 of Slit 3 protein.

EP 3 950 707 A1

[FIG. 1]

## PC DNA 3.1 (+) – SP Cystatin S – HSA(pro-) – H Slit3 LRR D2 – FLAG protein sequence

MARPLCTLLLLMATLAGALADAHKSEVAHRFKDLGEENFKALVLIAFAQYLQQCPFEDHVKLVNEVTEFAKTCVADESAENCDKSLHTLFGDKLCTVATLRETYG
EMADCCAKQEPERNECFLQHKDDNPNLPRLVRPEVDVMCTAFHDNEETFLKKYLYEIARRHPYFYAPELLFFAKRYKAAFTECCQAADKAACLLPKLDELRDE
GKASSAKQRLKCASLQKFGERAFKAWAVARLSQRFPKAEFAEVSKLVTDLTKVHTECCHGDLLECADDRADLAKYICENQDSISSKLKECCEKPLLEKSHCIAE
VENDEMPADLPSLAADFVESKDVCKNYAEAKDVFLGMFLYEYARRHPDYSVVLLLRLAKTYETTLEKCCAAADPHECYAKVFDEFKPLVEEPQNLIKQNCELFE
QLGEYKFQNALLVRYTKKVPQVSTPTLVEVSRNLGKVGSKCCKHPEAKRMPCAEDYLSVVLNQLCVLHEKTPVSDRVTKCCTESLVNRRPCFSALEVDETYVPK
EFNAETFTFHADICTLSEKERQIKKQTALVELVKHKPKATKEQLKAVMDDFAAFVEKCCKADDKETCFAEEGKKLVAASQAALGLISCPSPCTCSNNIVDCRGK
GLMEIPANLPEGIVEIRLEQNSIKAIPAGAFTQYKKLKRIDISKNQISDIAPDAFQGLKSLTSLVLYGNKITEIAKGLFDGLVSLQLLLLNANKINCLRVNTFQDLQNLN
LLSLYDNKLQTISKGLFAPLQSIQTLHLAQNPFVCDCHLKWLADYLQDNPIETSGARCSSPRRLANKRISQIKSKKFRCS DYKDDDDK* (SEQ ID NO: 9)

| SP | Cystatin S | 1-20AA (all 141AA) |
| --- | --- | --- |

| | HSA | 25-609AA (all 609AA) |
| --- | --- | --- |

| | LRR D2 | SLIT3 | 278-486AA(all 1523AA) |
| --- | --- | --- | --- |

| SP | HSA | LRR D2 | |
| --- | --- | --- | --- |
| 20AA | 585AA | 209AA | 9AA |

| 1 | Cystatin S | 2 | HSA(pro-) | 3 | H Slit3 LRR D2 | 4 | FLAG | * | Stop condon |
| --- | --- | --- | --- | --- | --- | --- | --- | --- | --- |

EP 3 950 707 A1

SEQUENCE LISTING

<110>   DAEWOONG PHARMACEUTICAL CO., LTD.

<120>   Composition for preventing or treating muscle related disease
        comprising HSA-conjugated Slit3 LRRD2

<130>   1067120

<150>   KR 10-2019-0023375
<151>   2019-02-27

<160>   18

<170>   PatentIn version 3.2

<210>   1
<211>   20
<212>   PRT
<213>   Artificial

<220>
<223>   SP Cystatin S

<400>   1

Met Ala Arg Pro Leu Cys Thr Leu Leu Leu Leu Met Ala Thr Leu Ala
1               5                   10                  15

Gly Ala Leu Ala
            20

<210>   2
<211>   585
<212>   PRT
<213>   Artificial

<220>
<223>   HSA

<400>   2

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
            35                  40                  45

Phe Ala Lys Thr Cys Val Ala Asp Glu Ser Ala Glu Asn Cys Asp Lys
        50                  55                  60

Ser Leu His Thr Leu Phe Gly Asp Lys Leu Cys Thr Val Ala Thr Leu
65                  70                  75                  80

```
Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145             150                 155                 160

Tyr Lys Ala Ala Phe Thr Glu Cys Cys Gln Ala Ala Asp Lys Ala Ala
            165                 170                 175

Cys Leu Leu Pro Lys Leu Asp Glu Leu Arg Asp Glu Gly Lys Ala Ser
        180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
            245                 250                 255

Arg Ala Asp Leu Ala Lys Tyr Ile Cys Glu Asn Gln Asp Ser Ile Ser
        260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335
```

```
Arg His Pro Asp Tyr Ser Val Val Leu Leu Leu Arg Leu Ala Lys Thr
            340             345             350

Tyr Glu Thr Thr Leu Glu Lys Cys Cys Ala Ala Ala Asp Pro His Glu
            355             360             365

Cys Tyr Ala Lys Val Phe Asp Glu Phe Lys Pro Leu Val Glu Glu Pro
            370             375             380

Gln Asn Leu Ile Lys Gln Asn Cys Glu Leu Phe Glu Gln Leu Gly Glu
385             390             395             400

Tyr Lys Phe Gln Asn Ala Leu Leu Val Arg Tyr Thr Lys Lys Val Pro
                405             410             415

Gln Val Ser Thr Pro Thr Leu Val Glu Val Ser Arg Asn Leu Gly Lys
            420             425             430

Val Gly Ser Lys Cys Cys Lys His Pro Glu Ala Lys Arg Met Pro Cys
            435             440             445

Ala Glu Asp Tyr Leu Ser Val Val Leu Asn Gln Leu Cys Val Leu His
            450             455             460

Glu Lys Thr Pro Val Ser Asp Arg Val Thr Lys Cys Cys Thr Glu Ser
465             470             475             480

Leu Val Asn Arg Arg Pro Cys Phe Ser Ala Leu Glu Val Asp Glu Thr
                485             490             495

Tyr Val Pro Lys Glu Phe Asn Ala Glu Thr Phe Thr Phe His Ala Asp
                500             505             510

Ile Cys Thr Leu Ser Glu Lys Glu Arg Gln Ile Lys Lys Gln Thr Ala
            515             520             525

Leu Val Glu Leu Val Lys His Lys Pro Lys Ala Thr Lys Glu Gln Leu
            530             535             540

Lys Ala Val Met Asp Asp Phe Ala Ala Phe Val Glu Lys Cys Cys Lys
545             550             555             560

Ala Asp Asp Lys Glu Thr Cys Phe Ala Glu Glu Gly Lys Lys Leu Val
                565             570             575

Ala Ala Ser Gln Ala Ala Leu Gly Leu
```

580                           585

<210>   3
<211>   209
<212>   PRT
<213>   Artificial

<220>
<223>   Slit3 LRRD2

<400>   3

Ile Ser Cys Pro Ser Pro Cys Thr Cys Ser Asn Asn Ile Val Asp Cys
1               5                   10                  15


Arg Gly Lys Gly Leu Met Glu Ile Pro Ala Asn Leu Pro Glu Gly Ile
            20                  25                  30


Val Glu Ile Arg Leu Glu Gln Asn Ser Ile Lys Ala Ile Pro Ala Gly
            35                  40                  45


Ala Phe Thr Gln Tyr Lys Lys Leu Lys Arg Ile Asp Ile Ser Lys Asn
        50                  55                  60


Gln Ile Ser Asp Ile Ala Pro Asp Ala Phe Gln Gly Leu Lys Ser Leu
65                  70                  75                  80


Thr Ser Leu Val Leu Tyr Gly Asn Lys Ile Thr Glu Ile Ala Lys Gly
                85                  90                  95


Leu Phe Asp Gly Leu Val Ser Leu Gln Leu Leu Leu Leu Asn Ala Asn
            100                 105                 110


Lys Ile Asn Cys Leu Arg Val Asn Thr Phe Gln Asp Leu Gln Asn Leu
        115                 120                 125


Asn Leu Leu Ser Leu Tyr Asp Asn Lys Leu Gln Thr Ile Ser Lys Gly
        130                 135                 140


Leu Phe Ala Pro Leu Gln Ser Ile Gln Thr Leu His Leu Ala Gln Asn
145                 150                 155                 160


Pro Phe Val Cys Asp Cys His Leu Lys Trp Leu Ala Asp Tyr Leu Gln
            165                 170                 175


Asp Asn Pro Ile Glu Thr Ser Gly Ala Arg Cys Ser Ser Pro Arg Arg
            180                 185                 190


Leu Ala Asn Lys Arg Ile Ser Gln Ile Lys Ser Lys Lys Phe Arg Cys
            195                 200                 205

32

Ser

<210> 4
<211> 8
<212> PRT
<213> Artificial

<220>
<223> FLAG

<400> 4

Asp Tyr Lys Asp Asp Asp Asp Lys
1               5


<210> 5
<211> 5
<212> PRT
<213> Artificial

<220>
<223> GS linker


<220>
<221> REPEAT
<222> (1)..(5)
<223> The amino acid residues at Position 1 to Postion 5 are repeated 1
      to 10 times.

<400> 5

Gly Gly Gly Gly Ser
1               5


<210> 6
<211> 15
<212> PRT
<213> Artificial

<220>
<223> GS linker

<400> 6

Gly Gly Gly Gly Ser Gly Gly Gly Gly Ser Gly Gly Gly Gly Ser
1               5                   10                  15


<210> 7
<211> 681
<212> PRT
<213> Artificial

<220>
<223> LRRD2-1

<400> 7

Met Ala Arg Pro Leu Cys Thr Leu Leu Leu Leu Met Ala Thr Leu Ala
1               5                   10                  15

Gly Ala Leu Ala Asp Ala His Lys Ser Glu Val Ala His Arg Phe Lys
            20                  25                  30

Asp Leu Gly Glu Glu Asn Phe Lys Ala Leu Val Leu Ile Ala Phe Ala
            35                  40                  45

Gln Tyr Leu Gln Gln Cys Pro Phe Glu Asp His Val Lys Leu Val Asn
        50                  55                  60

Glu Val Thr Glu Phe Ala Lys Thr Cys Val Ala Asp Glu Ser Ala Glu
65                  70                  75                  80

Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
            100                 105                 110

Lys Gln Glu Pro Glu Arg Asn Glu Cys Phe Leu Gln His Lys Asp Asp
        115                 120                 125

Asn Pro Asn Leu Pro Arg Leu Val Arg Pro Glu Val Asp Val Met Cys
        130                 135                 140

Thr Ala Phe His Asp Asn Glu Glu Thr Phe Leu Lys Lys Tyr Leu Tyr
145                 150                 155                 160

Glu Ile Ala Arg Arg His Pro Tyr Phe Tyr Ala Pro Glu Leu Leu Phe
            165                 170                 175

Phe Ala Lys Arg Tyr Lys Ala Ala Phe Thr Glu Cys Cys Gln Ala Ala
            180                 185                 190

Asp Lys Ala Ala Cys Leu Leu Pro Lys Leu Asp Glu Leu Arg Asp Glu
            195                 200                 205

Gly Lys Ala Ser Ser Ala Lys Gln Arg Leu Lys Cys Ala Ser Leu Gln
        210                 215                 220

Lys Phe Gly Glu Arg Ala Phe Lys Ala Trp Ala Val Ala Arg Leu Ser
225                 230                 235                 240

Gln Arg Phe Pro Lys Ala Glu Phe Ala Glu Val Ser Lys Leu Val Thr

```
                        245                         250                         255

      Asp Leu Thr Lys Val His Thr Glu Cys Cys His Gly Asp Leu Leu Glu
                  260                 265                 270

      Cys Ala Asp Asp Arg Ala Asp Leu Ala Lys Tyr Ile Cys Glu Asn Gln
              275                 280                 285

      Asp Ser Ile Ser Ser Lys Leu Lys Glu Cys Cys Glu Lys Pro Leu Leu
          290                 295                 300

      Glu Lys Ser His Cys Ile Ala Glu Val Glu Asn Asp Glu Met Pro Ala
      305                 310                 315                 320

      Asp Leu Pro Ser Leu Ala Ala Asp Phe Val Glu Ser Lys Asp Val Cys
                  325                 330                 335

      Lys Asn Tyr Ala Glu Ala Lys Asp Val Phe Leu Gly Met Phe Leu Tyr
                  340                 345                 350

      Glu Tyr Ala Arg Arg His Pro Asp Tyr Ser Val Val Leu Leu Leu Arg
                  355                 360                 365

      Leu Ala Lys Thr Tyr Glu Thr Thr Leu Glu Lys Cys Cys Ala Ala Ala
          370                 375                 380

      Asp Pro His Glu Cys Tyr Ala Lys Val Phe Asp Glu Phe Lys Pro Leu
      385                 390                 395                 400

      Val Glu Glu Pro Gln Asn Leu Ile Lys Gln Asn Cys Glu Leu Phe Glu
                  405                 410                 415

      Gln Leu Gly Glu Tyr Lys Phe Gln Asn Ala Leu Leu Val Arg Tyr Thr
                  420                 425                 430

      Lys Lys Val Pro Gln Val Ser Thr Pro Thr Leu Val Glu Val Ser Arg
                  435                 440                 445

      Asn Leu Gly Lys Val Gly Ser Lys Cys Cys Lys His Pro Glu Ala Lys
          450                 455                 460

      Arg Met Pro Cys Ala Glu Asp Tyr Leu Ser Val Val Leu Asn Gln Leu
      465                 470                 475                 480

      Cys Val Leu His Glu Lys Thr Pro Val Ser Asp Arg Val Thr Lys Cys
                  485                 490                 495
```

```
Cys Thr Glu Ser Leu Val Asn Arg Arg Pro Cys Phe Ser Ala Leu Glu
        500             505             510

Val Asp Glu Thr Tyr Val Pro Lys Glu Phe Asn Ala Glu Thr Phe Thr
        515             520             525

Phe His Ala Asp Ile Cys Thr Leu Ser Glu Lys Glu Arg Gln Ile Lys
        530             535             540

Lys Gln Thr Ala Leu Val Glu Leu Val Lys His Lys Pro Lys Ala Thr
545             550             555             560

Lys Glu Gln Leu Lys Ala Val Met Asp Asp Phe Ala Ala Phe Val Glu
                565             570             575

Lys Cys Cys Lys Ala Asp Asp Lys Glu Thr Cys Phe Ala Glu Glu Gly
            580             585             590

Lys Lys Leu Val Ala Ala Ser Gln Ala Ala Leu Gly Leu Leu Thr Ser
        595             600             605

Leu Val Leu Tyr Gly Asn Lys Ile Thr Glu Ile Ala Lys Gly Leu Phe
        610             615             620

Asp Gly Leu Val Ser Leu Gln Leu Leu Leu Leu Asn Ala Asn Lys Ile
625             630             635             640

Asn Cys Leu Arg Val Asn Thr Phe Gln Asp Leu Gln Asn Leu Asn Leu
            645             650             655

Leu Ser Leu Tyr Asp Asn Lys Leu Gln Thr Ile Ser Lys Gly Leu Phe
        660             665             670

Ala Asp Tyr Lys Asp Asp Asp Lys
        675             680
```

```
<210>   8
<211>   743
<212>   PRT
<213>   Artificial

<220>
<223>   LRRD2-2

<400>   8

Met Ala Arg Pro Leu Cys Thr Leu Leu Leu Met Ala Thr Leu Ala
1               5               10              15

Gly Ala Leu Ala Asp Ala His Lys Ser Glu Val Ala His Arg Phe Lys
```

|  | 20 |  | 25 |  | 30 |  |
|---|---|---|---|---|---|---|

Asp Leu Gly Glu Glu Asn Phe Lys Ala Leu Val Leu Ile Ala Phe Ala
          35               40               45

Gln Tyr Leu Gln Gln Cys Pro Phe Glu Asp His Val Lys Leu Val Asn
          50               55               60

Glu Val Thr Glu Phe Ala Lys Thr Cys Val Ala Asp Glu Ser Ala Glu
65                   70               75               80

Asn Cys Asp Lys Ser Leu His Thr Leu Phe Gly Asp Lys Leu Cys Thr
             85               90               95

Val Ala Thr Leu Arg Glu Thr Tyr Gly Glu Met Ala Asp Cys Cys Ala
          100             105            110

Lys Gln Glu Pro Glu Arg Asn Glu Cys Phe Leu Gln His Lys Asp Asp
          115             120            125

Asn Pro Asn Leu Pro Arg Leu Val Arg Pro Glu Val Asp Val Met Cys
          130             135            140

Thr Ala Phe His Asp Asn Glu Glu Thr Phe Leu Lys Lys Tyr Leu Tyr
145                   150               155           160

Glu Ile Ala Arg Arg His Pro Tyr Phe Tyr Ala Pro Glu Leu Leu Phe
          165             170           175

Phe Ala Lys Arg Tyr Lys Ala Ala Phe Thr Glu Cys Cys Gln Ala Ala
          180             185           190

Asp Lys Ala Ala Cys Leu Leu Pro Lys Leu Asp Glu Leu Arg Asp Glu
          195             200           205

Gly Lys Ala Ser Ser Ala Lys Gln Arg Leu Lys Cys Ala Ser Leu Gln
          210             215           220

Lys Phe Gly Glu Arg Ala Phe Lys Ala Trp Ala Val Ala Arg Leu Ser
225                   230               235           240

Gln Arg Phe Pro Lys Ala Glu Phe Ala Glu Val Ser Lys Leu Val Thr
          245             250           255

Asp Leu Thr Lys Val His Thr Glu Cys Cys His Gly Asp Leu Leu Glu
          260             265           270

Cys Ala Asp Asp Arg Ala Asp Leu Ala Lys Tyr Ile Cys Glu Asn Gln
        275                 280                 285

Asp Ser Ile Ser Ser Lys Leu Lys Glu Cys Cys Glu Lys Pro Leu Leu
    290                 295                 300

Glu Lys Ser His Cys Ile Ala Glu Val Glu Asn Asp Glu Met Pro Ala
305                 310                 315                 320

Asp Leu Pro Ser Leu Ala Ala Asp Phe Val Glu Ser Lys Asp Val Cys
            325                 330                 335

Lys Asn Tyr Ala Glu Ala Lys Asp Val Phe Leu Gly Met Phe Leu Tyr
        340                 345                 350

Glu Tyr Ala Arg Arg His Pro Asp Tyr Ser Val Val Leu Leu Leu Arg
        355                 360                 365

Leu Ala Lys Thr Tyr Glu Thr Thr Leu Glu Lys Cys Cys Ala Ala Ala
    370                 375                 380

Asp Pro His Glu Cys Tyr Ala Lys Val Phe Asp Glu Phe Lys Pro Leu
385                 390                 395                 400

Val Glu Glu Pro Gln Asn Leu Ile Lys Gln Asn Cys Glu Leu Phe Glu
            405                 410                 415

Gln Leu Gly Glu Tyr Lys Phe Gln Asn Ala Leu Leu Val Arg Tyr Thr
        420                 425                 430

Lys Lys Val Pro Gln Val Ser Thr Pro Thr Leu Val Glu Val Ser Arg
        435                 440                 445

Asn Leu Gly Lys Val Gly Ser Lys Cys Cys Lys His Pro Glu Ala Lys
    450                 455                 460

Arg Met Pro Cys Ala Glu Asp Tyr Leu Ser Val Val Leu Asn Gln Leu
465                 470                 475                 480

Cys Val Leu His Glu Lys Thr Pro Val Ser Asp Arg Val Thr Lys Cys
            485                 490                 495

Cys Thr Glu Ser Leu Val Asn Arg Arg Pro Cys Phe Ser Ala Leu Glu
        500                 505                 510

Val Asp Glu Thr Tyr Val Pro Lys Glu Phe Asn Ala Glu Thr Phe Thr
        515                 520                 525

```
Phe His Ala Asp Ile Cys Thr Leu Ser Glu Lys Glu Arg Gln Ile Lys
    530                 535             540

Lys Gln Thr Ala Leu Val Glu Leu Val Lys His Lys Pro Lys Ala Thr
545                 550             555             560

Lys Glu Gln Leu Lys Ala Val Met Asp Asp Phe Ala Ala Phe Val Glu
                565             570             575

Lys Cys Cys Lys Ala Asp Asp Lys Glu Thr Cys Phe Ala Glu Glu Gly
            580             585             590

Lys Lys Leu Val Ala Ala Ser Gln Ala Ala Leu Gly Leu Ile Val Glu
        595             600             605

Ile Arg Leu Glu Gln Asn Ser Ile Lys Ala Ile Pro Ala Gly Ala Phe
    610             615             620

Thr Gln Tyr Lys Lys Leu Lys Arg Ile Asp Ile Ser Lys Asn Gln Ile
625             630             635             640

Ser Asp Ile Ala Pro Asp Ala Phe Gln Gly Leu Lys Ser Leu Thr Ser
            645             650             655

Leu Val Leu Tyr Gly Asn Lys Ile Thr Glu Ile Ala Lys Gly Leu Phe
            660             665             670

Asp Gly Leu Val Ser Leu Gln Leu Leu Leu Leu Asn Ala Asn Lys Ile
        675             680             685

Asn Cys Leu Arg Val Asn Thr Phe Gln Asp Leu Gln Asn Leu Asn Leu
    690             695             700

Leu Ser Leu Tyr Asp Asn Lys Leu Gln Thr Ile Ser Lys Gly Leu Phe
705             710             715             720

Ala Pro Leu Gln Ser Ile Gln Thr Leu His Leu Ala Gln Asn Pro Asp
            725             730             735

Tyr Lys Asp Asp Asp Asp Lys
            740
```

```
<210>  9
<211>  822
<212>  PRT
<213>  Artificial

<220>
```

<223>  LRRD2-3

<400>  9

Met Ala Arg Pro Leu Cys Thr Leu Leu Leu Leu Met Ala Thr Leu Ala
1               5                   10                  15

Gly Ala Leu Ala Asp Ala His Lys Ser Glu Val Ala His Arg Phe Lys
            20                  25                  30

Asp Leu Gly Glu Glu Asn Phe Lys Ala Leu Val Leu Ile Ala Phe Ala
            35                  40                  45

Gln Tyr Leu Gln Gln Cys Pro Phe Glu Asp His Val Lys Leu Val Asn
        50                  55                  60

Glu Val Thr Glu Phe Ala Lys Thr Cys Val Ala Asp Glu Ser Ala Glu
65                  70                  75                  80

Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
            100                 105                 110

Lys Gln Glu Pro Glu Arg Asn Glu Cys Phe Leu Gln His Lys Asp Asp
        115                 120                 125

Asn Pro Asn Leu Pro Arg Leu Val Arg Pro Glu Val Asp Val Met Cys
        130                 135                 140

Thr Ala Phe His Asp Asn Glu Glu Thr Phe Leu Lys Lys Tyr Leu Tyr
145                 150                 155                 160

Glu Ile Ala Arg Arg His Pro Tyr Phe Tyr Ala Pro Glu Leu Leu Phe
            165                 170                 175

Phe Ala Lys Arg Tyr Lys Ala Ala Phe Thr Glu Cys Cys Gln Ala Ala
            180                 185                 190

Asp Lys Ala Ala Cys Leu Leu Pro Lys Leu Asp Glu Leu Arg Asp Glu
        195                 200                 205

Gly Lys Ala Ser Ser Ala Lys Gln Arg Leu Lys Cys Ala Ser Leu Gln
        210                 215                 220

Lys Phe Gly Glu Arg Ala Phe Lys Ala Trp Ala Val Ala Arg Leu Ser
225                 230                 235                 240

```
Gln Arg Phe Pro Lys Ala Glu Phe Ala Glu Val Ser Lys Leu Val Thr
            245             250             255

Asp Leu Thr Lys Val His Thr Glu Cys Cys His Gly Asp Leu Leu Glu
            260             265             270

Cys Ala Asp Asp Arg Ala Asp Leu Ala Lys Tyr Ile Cys Glu Asn Gln
            275             280             285

Asp Ser Ile Ser Ser Lys Leu Lys Glu Cys Cys Glu Lys Pro Leu Leu
            290             295             300

Glu Lys Ser His Cys Ile Ala Glu Val Glu Asn Asp Glu Met Pro Ala
305             310             315             320

Asp Leu Pro Ser Leu Ala Ala Asp Phe Val Glu Ser Lys Asp Val Cys
            325             330             335

Lys Asn Tyr Ala Glu Ala Lys Asp Val Phe Leu Gly Met Phe Leu Tyr
            340             345             350

Glu Tyr Ala Arg Arg His Pro Asp Tyr Ser Val Val Leu Leu Leu Arg
            355             360             365

Leu Ala Lys Thr Tyr Glu Thr Thr Leu Glu Lys Cys Cys Ala Ala Ala
            370             375             380

Asp Pro His Glu Cys Tyr Ala Lys Val Phe Asp Glu Phe Lys Pro Leu
385             390             395             400

Val Glu Glu Pro Gln Asn Leu Ile Lys Gln Asn Cys Glu Leu Phe Glu
            405             410             415

Gln Leu Gly Glu Tyr Lys Phe Gln Asn Ala Leu Leu Val Arg Tyr Thr
            420             425             430

Lys Lys Val Pro Gln Val Ser Thr Pro Thr Leu Val Glu Val Ser Arg
            435             440             445

Asn Leu Gly Lys Val Gly Ser Lys Cys Cys Lys His Pro Glu Ala Lys
            450             455             460

Arg Met Pro Cys Ala Glu Asp Tyr Leu Ser Val Val Leu Asn Gln Leu
465             470             475             480

Cys Val Leu His Glu Lys Thr Pro Val Ser Asp Arg Val Thr Lys Cys
            485             490             495
```

41

```
Cys Thr Glu Ser Leu Val Asn Arg Arg Pro Cys Phe Ser Ala Leu Glu
            500                 505                 510

Val Asp Glu Thr Tyr Val Pro Lys Glu Phe Asn Ala Glu Thr Phe Thr
            515                 520                 525

Phe His Ala Asp Ile Cys Thr Leu Ser Glu Lys Glu Arg Gln Ile Lys
            530                 535                 540

Lys Gln Thr Ala Leu Val Glu Leu Val Lys His Lys Pro Lys Ala Thr
545                 550                 555                 560

Lys Glu Gln Leu Lys Ala Val Met Asp Asp Phe Ala Ala Phe Val Glu
                565                 570                 575

Lys Cys Cys Lys Ala Asp Asp Lys Glu Thr Cys Phe Ala Glu Glu Gly
            580                 585                 590

Lys Lys Leu Val Ala Ala Ser Gln Ala Ala Leu Gly Leu Ile Ser Cys
            595                 600                 605

Pro Ser Pro Cys Thr Cys Ser Asn Asn Ile Val Asp Cys Arg Gly Lys
    610                 615                 620

Gly Leu Met Glu Ile Pro Ala Asn Leu Pro Glu Gly Ile Val Glu Ile
625                 630                 635                 640

Arg Leu Glu Gln Asn Ser Ile Lys Ala Ile Pro Ala Gly Ala Phe Thr
                645                 650                 655

Gln Tyr Lys Lys Leu Lys Arg Ile Asp Ile Ser Lys Asn Gln Ile Ser
            660                 665                 670

Asp Ile Ala Pro Asp Ala Phe Gln Gly Leu Lys Ser Leu Thr Ser Leu
            675                 680                 685

Val Leu Tyr Gly Asn Lys Ile Thr Glu Ile Ala Lys Gly Leu Phe Asp
            690                 695                 700

Gly Leu Val Ser Leu Gln Leu Leu Leu Leu Asn Ala Asn Lys Ile Asn
705                 710                 715                 720

Cys Leu Arg Val Asn Thr Phe Gln Asp Leu Gln Asn Leu Asn Leu Leu
                725                 730                 735

Ser Leu Tyr Asp Asn Lys Leu Gln Thr Ile Ser Lys Gly Leu Phe Ala
```

```
                  740                    745                         750

     Pro Leu Gln Ser Ile Gln Thr Leu His Leu Ala Gln Asn Pro Phe Val
             755                 760                 765

     Cys Asp Cys His Leu Lys Trp Leu Ala Asp Tyr Leu Gln Asp Asn Pro
             770                 775                 780

     Ile Glu Thr Ser Gly Ala Arg Cys Ser Ser Pro Arg Arg Leu Ala Asn
     785                 790                 795                     800

     Lys Arg Ile Ser Gln Ile Lys Ser Lys Lys Phe Arg Cys Ser Asp Tyr
                         805                 810                 815

     Lys Asp Asp Asp Asp Lys
                     820
```

<210> 10
<211> 696
<212> PRT
<213> Artificial

<220>
<223> LRRD2-4

<400> 10

```
     Met Ala Arg Pro Leu Cys Thr Leu Leu Leu Leu Met Ala Thr Leu Ala
     1               5                   10                  15

     Gly Ala Leu Ala Asp Ala His Lys Ser Glu Val Ala His Arg Phe Lys
                 20                  25                  30

     Asp Leu Gly Glu Glu Asn Phe Lys Ala Leu Val Leu Ile Ala Phe Ala
                 35                  40                  45

     Gln Tyr Leu Gln Gln Cys Pro Phe Glu Asp His Val Lys Leu Val Asn
             50                  55                  60

     Glu Val Thr Glu Phe Ala Lys Thr Cys Val Ala Asp Glu Ser Ala Glu
     65                  70                  75                  80

     Asn Cys Asp Lys Ser Leu His Thr Leu Phe Gly Asp Lys Leu Cys Thr
                     85                  90                  95

     Val Ala Thr Leu Arg Glu Thr Tyr Gly Glu Met Ala Asp Cys Cys Ala
                 100                 105                 110

     Lys Gln Glu Pro Glu Arg Asn Glu Cys Phe Leu Gln His Lys Asp Asp
             115                 120                 125
```

```
Asn Pro Asn Leu Pro Arg Leu Val Arg Pro Glu Val Asp Val Met Cys
    130             135             140

Thr Ala Phe His Asp Asn Glu Glu Thr Phe Leu Lys Lys Tyr Leu Tyr
    145             150             155             160

Glu Ile Ala Arg Arg His Pro Tyr Phe Tyr Ala Pro Glu Leu Leu Phe
                165             170             175

Phe Ala Lys Arg Tyr Lys Ala Ala Phe Thr Glu Cys Cys Gln Ala Ala
                180             185             190

Asp Lys Ala Ala Cys Leu Leu Pro Lys Leu Asp Glu Leu Arg Asp Glu
                195             200             205

Gly Lys Ala Ser Ser Ala Lys Gln Arg Leu Lys Cys Ala Ser Leu Gln
    210             215             220

Lys Phe Gly Glu Arg Ala Phe Lys Ala Trp Ala Val Ala Arg Leu Ser
    225             230             235             240

Gln Arg Phe Pro Lys Ala Glu Phe Ala Glu Val Ser Lys Leu Val Thr
                245             250             255

Asp Leu Thr Lys Val His Thr Glu Cys Cys His Gly Asp Leu Leu Glu
                260             265             270

Cys Ala Asp Asp Arg Ala Asp Leu Ala Lys Tyr Ile Cys Glu Asn Gln
                275             280             285

Asp Ser Ile Ser Ser Lys Leu Lys Glu Cys Cys Glu Lys Pro Leu Leu
    290             295             300

Glu Lys Ser His Cys Ile Ala Glu Val Glu Asn Asp Glu Met Pro Ala
    305             310             315             320

Asp Leu Pro Ser Leu Ala Ala Asp Phe Val Glu Ser Lys Asp Val Cys
                325             330             335

Lys Asn Tyr Ala Glu Ala Lys Asp Val Phe Leu Gly Met Phe Leu Tyr
                340             345             350

Glu Tyr Ala Arg Arg His Pro Asp Tyr Ser Val Val Leu Leu Leu Arg
                355             360             365

Leu Ala Lys Thr Tyr Glu Thr Thr Leu Glu Lys Cys Cys Ala Ala Ala
```

```
              370                        375                        380

         Asp Pro His Glu Cys Tyr Ala Lys Val Phe Asp Glu Phe Lys Pro Leu
         385                 390                 395                 400


         Val Glu Glu Pro Gln Asn Leu Ile Lys Gln Asn Cys Glu Leu Phe Glu
                         405                 410                 415


         Gln Leu Gly Glu Tyr Lys Phe Gln Asn Ala Leu Leu Val Arg Tyr Thr
                         420                 425                 430


         Lys Lys Val Pro Gln Val Ser Thr Pro Thr Leu Val Glu Val Ser Arg
                         435                 440                 445


         Asn Leu Gly Lys Val Gly Ser Lys Cys Cys Lys His Pro Glu Ala Lys
                 450                 455                 460


         Arg Met Pro Cys Ala Glu Asp Tyr Leu Ser Val Val Leu Asn Gln Leu
         465                 470                 475                 480


         Cys Val Leu His Glu Lys Thr Pro Val Ser Asp Arg Val Thr Lys Cys
                         485                 490                 495


         Cys Thr Glu Ser Leu Val Asn Arg Arg Pro Cys Phe Ser Ala Leu Glu
                     500                 505                 510


         Val Asp Glu Thr Tyr Val Pro Lys Glu Phe Asn Ala Glu Thr Phe Thr
                     515                 520                 525


         Phe His Ala Asp Ile Cys Thr Leu Ser Glu Lys Glu Arg Gln Ile Lys
                 530                 535                 540


         Lys Gln Thr Ala Leu Val Glu Leu Val Lys His Lys Pro Lys Ala Thr
         545                 550                 555                 560


         Lys Glu Gln Leu Lys Ala Val Met Asp Asp Phe Ala Ala Phe Val Glu
                         565                 570                 575


         Lys Cys Cys Lys Ala Asp Asp Lys Glu Thr Cys Phe Ala Glu Glu Gly
                     580                 585                 590


         Lys Lys Leu Val Ala Ala Ser Gln Ala Ala Leu Gly Leu Gly Gly Gly
                     595                 600                 605


         Gly Ser Gly Gly Gly Gly Ser Gly Gly Gly Gly Ser Leu Thr Ser Leu
             610                 615                 620
```

Val Leu Tyr Gly Asn Lys Ile Thr Glu Ile Ala Lys Gly Leu Phe Asp
625                 630                 635                 640

Gly Leu Val Ser Leu Gln Leu Leu Leu Leu Asn Ala Asn Lys Ile Asn
                    645                 650                 655

Cys Leu Arg Val Asn Thr Phe Gln Asp Leu Gln Asn Leu Asn Leu Leu
                660                 665                 670

Ser Leu Tyr Asp Asn Lys Leu Gln Thr Ile Ser Lys Gly Leu Phe Ala
                675                 680                 685

Asp Tyr Lys Asp Asp Asp Asp Lys
    690                 695

<210> 11
<211> 758
<212> PRT
<213> Artificial

<220>
<223> LRRD2-5

<400> 11

Met Ala Arg Pro Leu Cys Thr Leu Leu Leu Leu Met Ala Thr Leu Ala
1               5                   10                  15

Gly Ala Leu Ala Asp Ala His Lys Ser Glu Val Ala His Arg Phe Lys
                20                  25                  30

Asp Leu Gly Glu Glu Asn Phe Lys Ala Leu Val Leu Ile Ala Phe Ala
            35                  40                  45

Gln Tyr Leu Gln Gln Cys Pro Phe Glu Asp His Val Lys Leu Val Asn
    50                  55                  60

Glu Val Thr Glu Phe Ala Lys Thr Cys Val Ala Asp Glu Ser Ala Glu
65                  70                  75                  80

Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
                100                 105                 110

Lys Gln Glu Pro Glu Arg Asn Glu Cys Phe Leu Gln His Lys Asp Asp
            115                 120                 125

Asn Pro Asn Leu Pro Arg Leu Val Arg Pro Glu Val Asp Val Met Cys

```
            130                    135                      140


Thr Ala Phe His Asp Asn Glu Glu Thr Phe Leu Lys Lys Tyr Leu Tyr
145                 150                 155                 160


Glu Ile Ala Arg Arg His Pro Tyr Phe Tyr Ala Pro Glu Leu Leu Phe
                165                 170                 175


Phe Ala Lys Arg Tyr Lys Ala Ala Phe Thr Glu Cys Cys Gln Ala Ala
            180                 185                 190


Asp Lys Ala Ala Cys Leu Leu Pro Lys Leu Asp Glu Leu Arg Asp Glu
            195                 200                 205


Gly Lys Ala Ser Ser Ala Lys Gln Arg Leu Lys Cys Ala Ser Leu Gln
    210                 215                 220


Lys Phe Gly Glu Arg Ala Phe Lys Ala Trp Ala Val Ala Arg Leu Ser
225                 230                 235                 240


Gln Arg Phe Pro Lys Ala Glu Phe Ala Glu Val Ser Lys Leu Val Thr
                245                 250                 255


Asp Leu Thr Lys Val His Thr Glu Cys Cys His Gly Asp Leu Leu Glu
            260                 265                 270


Cys Ala Asp Asp Arg Ala Asp Leu Ala Lys Tyr Ile Cys Glu Asn Gln
            275                 280                 285


Asp Ser Ile Ser Ser Lys Leu Lys Glu Cys Cys Glu Lys Pro Leu Leu
    290                 295                 300


Glu Lys Ser His Cys Ile Ala Glu Val Glu Asn Asp Glu Met Pro Ala
305                 310                 315                 320


Asp Leu Pro Ser Leu Ala Ala Asp Phe Val Glu Ser Lys Asp Val Cys
                325                 330                 335


Lys Asn Tyr Ala Glu Ala Lys Asp Val Phe Leu Gly Met Phe Leu Tyr
            340                 345                 350


Glu Tyr Ala Arg Arg His Pro Asp Tyr Ser Val Val Leu Leu Leu Arg
            355                 360                 365


Leu Ala Lys Thr Tyr Glu Thr Thr Leu Glu Lys Cys Cys Ala Ala Ala
    370                 375                 380
```

47

Asp Pro His Glu Cys Tyr Ala Lys Val Phe Asp Glu Phe Lys Pro Leu
385                 390                 395                 400

Val Glu Glu Pro Gln Asn Leu Ile Lys Gln Asn Cys Glu Leu Phe Glu
                405                 410                 415

Gln Leu Gly Glu Tyr Lys Phe Gln Asn Ala Leu Leu Val Arg Tyr Thr
                420                 425                 430

Lys Lys Val Pro Gln Val Ser Thr Pro Thr Leu Val Glu Val Ser Arg
        435                 440                 445

Asn Leu Gly Lys Val Gly Ser Lys Cys Cys Lys His Pro Glu Ala Lys
    450                 455                 460

Arg Met Pro Cys Ala Glu Asp Tyr Leu Ser Val Val Leu Asn Gln Leu
465                 470                 475                 480

Cys Val Leu His Glu Lys Thr Pro Val Ser Asp Arg Val Thr Lys Cys
                485                 490                 495

Cys Thr Glu Ser Leu Val Asn Arg Arg Pro Cys Phe Ser Ala Leu Glu
                500                 505                 510

Val Asp Glu Thr Tyr Val Pro Lys Glu Phe Asn Ala Glu Thr Phe Thr
                515                 520                 525

Phe His Ala Asp Ile Cys Thr Leu Ser Glu Lys Glu Arg Gln Ile Lys
    530                 535                 540

Lys Gln Thr Ala Leu Val Glu Leu Val Lys His Lys Pro Lys Ala Thr
545                 550                 555                 560

Lys Glu Gln Leu Lys Ala Val Met Asp Asp Phe Ala Ala Phe Val Glu
                565                 570                 575

Lys Cys Cys Lys Ala Asp Asp Lys Glu Thr Cys Phe Ala Glu Glu Gly
        580                 585                 590

Lys Lys Leu Val Ala Ala Ser Gln Ala Ala Leu Gly Leu Gly Gly Gly
        595                 600                 605

Gly Ser Gly Gly Gly Gly Ser Gly Gly Gly Gly Ser Ile Val Glu Ile
    610                 615                 620

Arg Leu Glu Gln Asn Ser Ile Lys Ala Ile Pro Ala Gly Ala Phe Thr
625                 630                 635                 640

Gln Tyr Lys Lys Leu Lys Arg Ile Asp Ile Ser Lys Asn Gln Ile Ser
                645             650             655

Asp Ile Ala Pro Asp Ala Phe Gln Gly Leu Lys Ser Leu Thr Ser Leu
            660             665             670

Val Leu Tyr Gly Asn Lys Ile Thr Glu Ile Ala Lys Gly Leu Phe Asp
            675             680             685

Gly Leu Val Ser Leu Gln Leu Leu Leu Leu Asn Ala Asn Lys Ile Asn
    690             695             700

Cys Leu Arg Val Asn Thr Phe Gln Asp Leu Gln Asn Leu Asn Leu Leu
705             710             715             720

Ser Leu Tyr Asp Asn Lys Leu Gln Thr Ile Ser Lys Gly Leu Phe Ala
            725             730             735

Pro Leu Gln Ser Ile Gln Thr Leu His Leu Ala Gln Asn Pro Asp Tyr
            740             745             750

Lys Asp Asp Asp Asp Lys
            755

<210> 12
<211> 837
<212> PRT
<213> Artificial

<220>
<223> LRRD2-6

<400> 12

Met Ala Arg Pro Leu Cys Thr Leu Leu Leu Met Ala Thr Leu Ala
1               5               10              15

Gly Ala Leu Ala Asp Ala His Lys Ser Glu Val Ala His Arg Phe Lys
            20              25              30

Asp Leu Gly Glu Glu Asn Phe Lys Ala Leu Val Leu Ile Ala Phe Ala
            35              40              45

Gln Tyr Leu Gln Gln Cys Pro Phe Glu Asp His Val Lys Leu Val Asn
    50              55              60

Glu Val Thr Glu Phe Ala Lys Thr Cys Val Ala Asp Glu Ser Ala Glu
65              70              75              80

49

Asn Cys Asp Lys Ser Leu His Thr Leu Phe Gly Asp Lys Leu Cys Thr
85                          90                      95

Val Ala Thr Leu Arg Glu Thr Tyr Gly Glu Met Ala Asp Cys Cys Ala
100                         105                     110

Lys Gln Glu Pro Glu Arg Asn Glu Cys Phe Leu Gln His Lys Asp Asp
115                         120                     125

Asn Pro Asn Leu Pro Arg Leu Val Arg Pro Glu Val Asp Val Met Cys
130                         135                     140

Thr Ala Phe His Asp Asn Glu Glu Thr Phe Leu Lys Lys Tyr Leu Tyr
145                         150                     155                     160

Glu Ile Ala Arg Arg His Pro Tyr Phe Tyr Ala Pro Glu Leu Leu Phe
165                         170                     175

Phe Ala Lys Arg Tyr Lys Ala Ala Phe Thr Glu Cys Cys Gln Ala Ala
180                         185                     190

Asp Lys Ala Ala Cys Leu Leu Pro Lys Leu Asp Glu Leu Arg Asp Glu
195                         200                     205

Gly Lys Ala Ser Ser Ala Lys Gln Arg Leu Lys Cys Ala Ser Leu Gln
210                         215                     220

Lys Phe Gly Glu Arg Ala Phe Lys Ala Trp Ala Val Ala Arg Leu Ser
225                         230                     235                     240

Gln Arg Phe Pro Lys Ala Glu Phe Ala Glu Val Ser Lys Leu Val Thr
245                         250                     255

Asp Leu Thr Lys Val His Thr Glu Cys Cys His Gly Asp Leu Leu Glu
260                         265                     270

Cys Ala Asp Asp Arg Ala Asp Leu Ala Lys Tyr Ile Cys Glu Asn Gln
275                         280                     285

Asp Ser Ile Ser Ser Lys Leu Lys Glu Cys Cys Glu Lys Pro Leu Leu
290                         295                     300

Glu Lys Ser His Cys Ile Ala Glu Val Glu Asn Asp Glu Met Pro Ala
305                         310                     315                     320

Asp Leu Pro Ser Leu Ala Ala Asp Phe Val Glu Ser Lys Asp Val Cys
325                         330                     335

Lys Asn Tyr Ala Glu Ala Lys Asp Val Phe Leu Gly Met Phe Leu Tyr
           340                 345                  350

Glu Tyr Ala Arg Arg His Pro Asp Tyr Ser Val Val Leu Leu Leu Arg
           355                 360                  365

Leu Ala Lys Thr Tyr Glu Thr Thr Leu Glu Lys Cys Cys Ala Ala Ala
           370                 375                  380

Asp Pro His Glu Cys Tyr Ala Lys Val Phe Asp Glu Phe Lys Pro Leu
385                 390                 395              400

Val Glu Glu Pro Gln Asn Leu Ile Lys Gln Asn Cys Glu Leu Phe Glu
           405                 410                  415

Gln Leu Gly Glu Tyr Lys Phe Gln Asn Ala Leu Leu Val Arg Tyr Thr
           420                 425                  430

Lys Lys Val Pro Gln Val Ser Thr Pro Thr Leu Val Glu Val Ser Arg
           435                 440                  445

Asn Leu Gly Lys Val Gly Ser Lys Cys Cys Lys His Pro Glu Ala Lys
           450                 455                  460

Arg Met Pro Cys Ala Glu Asp Tyr Leu Ser Val Val Leu Asn Gln Leu
465                 470                 475              480

Cys Val Leu His Glu Lys Thr Pro Val Ser Asp Arg Val Thr Lys Cys
           485                 490                  495

Cys Thr Glu Ser Leu Val Asn Arg Arg Pro Cys Phe Ser Ala Leu Glu
           500                 505                  510

Val Asp Glu Thr Tyr Val Pro Lys Glu Phe Asn Ala Glu Thr Phe Thr
           515                 520                  525

Phe His Ala Asp Ile Cys Thr Leu Ser Glu Lys Glu Arg Gln Ile Lys
           530                 535                  540

Lys Gln Thr Ala Leu Val Glu Leu Val Lys His Lys Pro Lys Ala Thr
545                 550                 555              560

Lys Glu Gln Leu Lys Ala Val Met Asp Asp Phe Ala Ala Phe Val Glu
           565                 570                  575

Lys Cys Cys Lys Ala Asp Asp Lys Glu Thr Cys Phe Ala Glu Glu Gly
           580                 585                  590

Lys Lys Leu Val Ala Ala Ser Gln Ala Ala Leu Gly Leu Gly Gly Gly
        595                 600             605

Gly Ser Gly Gly Gly Gly Ser Gly Gly Gly Gly Ser Ile Ser Cys Pro
    610                 615                 620

Ser Pro Cys Thr Cys Ser Asn Asn Ile Val Asp Cys Arg Gly Lys Gly
625                 630                 635                 640

Leu Met Glu Ile Pro Ala Asn Leu Pro Glu Gly Ile Val Glu Ile Arg
                645                 650                 655

Leu Glu Gln Asn Ser Ile Lys Ala Ile Pro Ala Gly Ala Phe Thr Gln
                660                 665                 670

Tyr Lys Lys Leu Lys Arg Ile Asp Ile Ser Lys Asn Gln Ile Ser Asp
        675                 680                 685

Ile Ala Pro Asp Ala Phe Gln Gly Leu Lys Ser Leu Thr Ser Leu Val
    690                 695                 700

Leu Tyr Gly Asn Lys Ile Thr Glu Ile Ala Lys Gly Leu Phe Asp Gly
705                 710                 715                 720

Leu Val Ser Leu Gln Leu Leu Leu Leu Asn Ala Asn Lys Ile Asn Cys
                725                 730                 735

Leu Arg Val Asn Thr Phe Gln Asp Leu Gln Asn Leu Asn Leu Leu Ser
            740                 745                 750

Leu Tyr Asp Asn Lys Leu Gln Thr Ile Ser Lys Gly Leu Phe Ala Pro
        755                 760                 765

Leu Gln Ser Ile Gln Thr Leu His Leu Ala Gln Asn Pro Phe Val Cys
    770                 775                 780

Asp Cys His Leu Lys Trp Leu Ala Asp Tyr Leu Gln Asp Asn Pro Ile
785                 790                 795                 800

Glu Thr Ser Gly Ala Arg Cys Ser Ser Pro Arg Arg Leu Ala Asn Lys
                805                 810                 815

Arg Ile Ser Gln Ile Lys Ser Lys Lys Phe Arg Cys Ser Asp Tyr Lys
        820                 825                 830

Asp Asp Asp Asp Lys

835

```
<210>  13
<211>  696
<212>  PRT
<213>  Artificial

<220>
<223>  LRRD2-7

<400>  13
```

Met Ala Arg Pro Leu Cys Thr Leu Leu Leu Leu Met Ala Thr Leu Ala
1               5                   10                  15

Gly Ala Leu Ala Leu Thr Ser Leu Val Leu Tyr Gly Asn Lys Ile Thr
            20                  25                  30

Glu Ile Ala Lys Gly Leu Phe Asp Gly Leu Val Ser Leu Gln Leu Leu
        35                  40                  45

Leu Leu Asn Ala Asn Lys Ile Asn Cys Leu Arg Val Asn Thr Phe Gln
    50                  55                  60

Asp Leu Gln Asn Leu Asn Leu Leu Ser Leu Tyr Asp Asn Lys Leu Gln
65                  70                  75                  80

Thr Ile Ser Lys Gly Leu Phe Ala Asp Ala His Lys Ser Glu Val Ala
                85                  90                  95

His Arg Phe Lys Asp Leu Gly Glu Glu Asn Phe Lys Ala Leu Val Leu
            100                 105                 110

Ile Ala Phe Ala Gln Tyr Leu Gln Gln Cys Pro Phe Glu Asp His Val
            115                 120                 125

Lys Leu Val Asn Glu Val Thr Glu Phe Ala Lys Thr Cys Val Ala Asp
    130                 135                 140

Glu Ser Ala Glu Asn Cys Asp Lys Ser Leu His Thr Leu Phe Gly Asp
145                 150                 155                 160

Lys Leu Cys Thr Val Ala Thr Leu Arg Glu Thr Tyr Gly Glu Met Ala
                165                 170                 175

Asp Cys Cys Ala Lys Gln Glu Pro Glu Arg Asn Glu Cys Phe Leu Gln
            180                 185                 190

His Lys Asp Asp Asn Pro Asn Leu Pro Arg Leu Val Arg Pro Glu Val
            195                 200                 205

```
Asp Val Met Cys Thr Ala Phe His Asp Asn Glu Glu Thr Phe Leu Lys
    210             215             220

Lys Tyr Leu Tyr Glu Ile Ala Arg Arg His Pro Tyr Phe Tyr Ala Pro
225             230             235             240

Glu Leu Leu Phe Phe Ala Lys Arg Tyr Lys Ala Ala Phe Thr Glu Cys
            245             250             255

Cys Gln Ala Ala Asp Lys Ala Ala Cys Leu Leu Pro Lys Leu Asp Glu
        260             265             270

Leu Arg Asp Glu Gly Lys Ala Ser Ser Ala Lys Gln Arg Leu Lys Cys
        275             280             285

Ala Ser Leu Gln Lys Phe Gly Glu Arg Ala Phe Lys Ala Trp Ala Val
    290             295             300

Ala Arg Leu Ser Gln Arg Phe Pro Lys Ala Glu Phe Ala Glu Val Ser
305             310             315             320

Lys Leu Val Thr Asp Leu Thr Lys Val His Thr Glu Cys Cys His Gly
            325             330             335

Asp Leu Leu Glu Cys Ala Asp Asp Arg Ala Asp Leu Ala Lys Tyr Ile
        340             345             350

Cys Glu Asn Gln Asp Ser Ile Ser Ser Lys Leu Lys Glu Cys Cys Glu
        355             360             365

Lys Pro Leu Leu Glu Lys Ser His Cys Ile Ala Glu Val Glu Asn Asp
    370             375             380

Glu Met Pro Ala Asp Leu Pro Ser Leu Ala Ala Asp Phe Val Glu Ser
385             390             395             400

Lys Asp Val Cys Lys Asn Tyr Ala Glu Ala Lys Asp Val Phe Leu Gly
            405             410             415

Met Phe Leu Tyr Glu Tyr Ala Arg Arg His Pro Asp Tyr Ser Val Val
            420             425             430

Leu Leu Leu Arg Leu Ala Lys Thr Tyr Glu Thr Thr Leu Glu Lys Cys
        435             440             445

Cys Ala Ala Ala Asp Pro His Glu Cys Tyr Ala Lys Val Phe Asp Glu
```

```
                450                      455                          460


        Phe Lys Pro Leu Val Glu Glu Pro Gln Asn Leu Ile Lys Gln Asn Cys
        465             470             475                 480


        Glu Leu Phe Glu Gln Leu Gly Glu Tyr Lys Phe Gln Asn Ala Leu Leu
                    485             490                 495


        Val Arg Tyr Thr Lys Lys Val Pro Gln Val Ser Thr Pro Thr Leu Val
                    500             505             510


        Glu Val Ser Arg Asn Leu Gly Lys Val Gly Ser Lys Cys Cys Lys His
                    515             520             525


        Pro Glu Ala Lys Arg Met Pro Cys Ala Glu Asp Tyr Leu Ser Val Val
            530             535             540


        Leu Asn Gln Leu Cys Val Leu His Glu Lys Thr Pro Val Ser Asp Arg
        545             550             555                 560


        Val Thr Lys Cys Cys Thr Glu Ser Leu Val Asn Arg Arg Pro Cys Phe
                    565             570             575


        Ser Ala Leu Glu Val Asp Glu Thr Tyr Val Pro Lys Glu Phe Asn Ala
                    580             585             590


        Glu Thr Phe Thr Phe His Ala Asp Ile Cys Thr Leu Ser Glu Lys Glu
                    595             600             605


        Arg Gln Ile Lys Lys Gln Thr Ala Leu Val Glu Leu Val Lys His Lys
                    610             615             620


        Pro Lys Ala Thr Lys Glu Gln Leu Lys Ala Val Met Asp Asp Phe Ala
        625             630             635                 640


        Ala Phe Val Glu Lys Cys Cys Lys Ala Asp Asp Lys Glu Thr Cys Phe
                    645             650             655


        Ala Glu Glu Gly Lys Lys Leu Val Ala Ala Ser Gln Ala Ala Leu Gly
                    660             665             670


        Leu Gly Gly Gly Gly Ser Gly Gly Gly Ser Gly Gly Gly Gly Ser
                    675             680             685


        Asp Tyr Lys Asp Asp Asp Asp Lys
            690             695
```

```
<210>   14
<211>   758
<212>   PRT
<213>   Artificial

<220>
<223>   LRRD2-8

<400>   14
```

Met Ala Arg Pro Leu Cys Thr Leu Leu Leu Leu Met Ala Thr Leu Ala
1               5                   10                  15

Gly Ala Leu Ala Ile Val Glu Ile Arg Leu Glu Gln Asn Ser Ile Lys
            20                  25                  30

Ala Ile Pro Ala Gly Ala Phe Thr Gln Tyr Lys Lys Leu Lys Arg Ile
        35                  40                  45

Asp Ile Ser Lys Asn Gln Ile Ser Asp Ile Ala Pro Asp Ala Phe Gln
        50                  55                  60

Gly Leu Lys Ser Leu Thr Ser Leu Val Leu Tyr Gly Asn Lys Ile Thr
65                  70                  75                  80

Glu Ile Ala Lys Gly Leu Phe Asp Gly Leu Val Ser Leu Gln Leu Leu
            85                  90                  95

Leu Leu Asn Ala Asn Lys Ile Asn Cys Leu Arg Val Asn Thr Phe Gln
            100                 105                 110

Asp Leu Gln Asn Leu Asn Leu Leu Ser Leu Tyr Asp Asn Lys Leu Gln
        115                 120                 125

Thr Ile Ser Lys Gly Leu Phe Ala Pro Leu Gln Ser Ile Gln Thr Leu
        130                 135                 140

His Leu Ala Gln Asn Pro Asp Ala His Lys Ser Glu Val Ala His Arg
145                 150                 155                 160

Phe Lys Asp Leu Gly Glu Glu Asn Phe Lys Ala Leu Val Leu Ile Ala
            165                 170                 175

Phe Ala Gln Tyr Leu Gln Gln Cys Pro Phe Glu Asp His Val Lys Leu
            180                 185                 190

Val Asn Glu Val Thr Glu Phe Ala Lys Thr Cys Val Ala Asp Glu Ser
        195                 200                 205

Ala Glu Asn Cys Asp Lys Ser Leu His Thr Leu Phe Gly Asp Lys Leu

```
                210                        215                        220


        Cys Thr Val Ala Thr Leu Arg Glu Thr Tyr Gly Glu Met Ala Asp Cys
        225                 230                 235                 240


        Cys Ala Lys Gln Glu Pro Glu Arg Asn Glu Cys Phe Leu Gln His Lys
                        245                 250                 255


        Asp Asp Asn Pro Asn Leu Pro Arg Leu Val Arg Pro Glu Val Asp Val
                        260                 265                 270


        Met Cys Thr Ala Phe His Asp Asn Glu Glu Thr Phe Leu Lys Lys Tyr
                        275                 280                 285


        Leu Tyr Glu Ile Ala Arg Arg His Pro Tyr Phe Tyr Ala Pro Glu Leu
                290                 295                 300


        Leu Phe Phe Ala Lys Arg Tyr Lys Ala Ala Phe Thr Glu Cys Cys Gln
        305                 310                 315                 320


        Ala Ala Asp Lys Ala Ala Cys Leu Leu Pro Lys Leu Asp Glu Leu Arg
                        325                 330                 335


        Asp Glu Gly Lys Ala Ser Ser Ala Lys Gln Arg Leu Lys Cys Ala Ser
                        340                 345                 350


        Leu Gln Lys Phe Gly Glu Arg Ala Phe Lys Ala Trp Ala Val Ala Arg
                        355                 360                 365


        Leu Ser Gln Arg Phe Pro Lys Ala Glu Phe Ala Glu Val Ser Lys Leu
                370                 375                 380


        Val Thr Asp Leu Thr Lys Val His Thr Glu Cys Cys His Gly Asp Leu
        385                 390                 395                 400


        Leu Glu Cys Ala Asp Asp Arg Ala Asp Leu Ala Lys Tyr Ile Cys Glu
                        405                 410                 415


        Asn Gln Asp Ser Ile Ser Ser Lys Leu Lys Glu Cys Cys Glu Lys Pro
                        420                 425                 430


        Leu Leu Glu Lys Ser His Cys Ile Ala Glu Val Glu Asn Asp Glu Met
                435                 440                 445


        Pro Ala Asp Leu Pro Ser Leu Ala Ala Asp Phe Val Glu Ser Lys Asp
                450                 455                 460
```

```
Val Cys Lys Asn Tyr Ala Glu Ala Lys Asp Val Phe Leu Gly Met Phe
465                 470                 475                 480


Leu Tyr Glu Tyr Ala Arg Arg His Pro Asp Tyr Ser Val Val Leu Leu
                485                 490                 495


Leu Arg Leu Ala Lys Thr Tyr Glu Thr Thr Leu Glu Lys Cys Cys Ala
            500                 505                 510


Ala Ala Asp Pro His Glu Cys Tyr Ala Lys Val Phe Asp Glu Phe Lys
        515                 520                 525


Pro Leu Val Glu Glu Pro Gln Asn Leu Ile Lys Gln Asn Cys Glu Leu
    530                 535                 540


Phe Glu Gln Leu Gly Glu Tyr Lys Phe Gln Asn Ala Leu Leu Val Arg
545                 550                 555                 560


Tyr Thr Lys Lys Val Pro Gln Val Ser Thr Pro Thr Leu Val Glu Val
                565                 570                 575


Ser Arg Asn Leu Gly Lys Val Gly Ser Lys Cys Cys Lys His Pro Glu
            580                 585                 590


Ala Lys Arg Met Pro Cys Ala Glu Asp Tyr Leu Ser Val Val Leu Asn
        595                 600                 605


Gln Leu Cys Val Leu His Glu Lys Thr Pro Val Ser Asp Arg Val Thr
    610                 615                 620


Lys Cys Cys Thr Glu Ser Leu Val Asn Arg Arg Pro Cys Phe Ser Ala
625                 630                 635                 640


Leu Glu Val Asp Glu Thr Tyr Val Pro Lys Glu Phe Asn Ala Glu Thr
                645                 650                 655


Phe Thr Phe His Ala Asp Ile Cys Thr Leu Ser Glu Lys Glu Arg Gln
            660                 665                 670


Ile Lys Lys Gln Thr Ala Leu Val Glu Leu Val Lys His Lys Pro Lys
            675                 680                 685


Ala Thr Lys Glu Gln Leu Lys Ala Val Met Asp Asp Phe Ala Ala Phe
        690                 695                 700


Val Glu Lys Cys Cys Lys Ala Asp Asp Lys Glu Thr Cys Phe Ala Glu
705                 710                 715                 720
```

58

EP 3 950 707 A1

Glu Gly Lys Lys Leu Val Ala Ala Ser Gln Ala Ala Leu Gly Leu Gly
                725             730             735

Gly Gly Gly Ser Gly Gly Gly Gly Ser Gly Gly Gly Gly Ser Asp Tyr
                740             745             750

Lys Asp Asp Asp Asp Lys
                755

<210> 15
<211> 837
<212> PRT
<213> Artificial

<220>
<223> LRRD2-9

<400> 15

Met Ala Arg Pro Leu Cys Thr Leu Leu Leu Met Ala Thr Leu Ala
1               5               10              15

Gly Ala Leu Ala Ile Ser Cys Pro Ser Pro Cys Thr Cys Ser Asn Asn
                20              25              30

Ile Val Asp Cys Arg Gly Lys Gly Leu Met Glu Ile Pro Ala Asn Leu
                35              40              45

Pro Glu Gly Ile Val Glu Ile Arg Leu Glu Gln Asn Ser Ile Lys Ala
        50              55              60

Ile Pro Ala Gly Ala Phe Thr Gln Tyr Lys Lys Leu Lys Arg Ile Asp
65              70              75              80

Ile Ser Lys Asn Gln Ile Ser Asp Ile Ala Pro Asp Ala Phe Gln Gly
                85              90              95

Leu Lys Ser Leu Thr Ser Leu Val Leu Tyr Gly Asn Lys Ile Thr Glu
                100             105             110

Ile Ala Lys Gly Leu Phe Asp Gly Leu Val Ser Leu Gln Leu Leu Leu
                115             120             125

Leu Asn Ala Asn Lys Ile Asn Cys Leu Arg Val Asn Thr Phe Gln Asp
        130             135             140

Leu Gln Asn Leu Asn Leu Leu Ser Leu Tyr Asp Asn Lys Leu Gln Thr
145             150             155             160

59

Ile Ser Lys Gly Leu Phe Ala Pro Leu Gln Ser Ile Gln Thr Leu His
165 170 175

Leu Ala Gln Asn Pro Phe Val Cys Asp Cys His Leu Lys Trp Leu Ala
180 185 190

Asp Tyr Leu Gln Asp Asn Pro Ile Glu Thr Ser Gly Ala Arg Cys Ser
195 200 205

Ser Pro Arg Arg Leu Ala Asn Lys Arg Ile Ser Gln Ile Lys Ser Lys
210 215 220

Lys Phe Arg Cys Ser Asp Ala His Lys Ser Glu Val Ala His Arg Phe
225 230 235 240

Lys Asp Leu Gly Glu Glu Asn Phe Lys Ala Leu Val Leu Ile Ala Phe
245 250 255

Ala Gln Tyr Leu Gln Gln Cys Pro Phe Glu Asp His Val Lys Leu Val
260 265 270

Asn Glu Val Thr Glu Phe Ala Lys Thr Cys Val Ala Asp Glu Ser Ala
275 280 285

Glu Asn Cys Asp Lys Ser Leu His Thr Leu Phe Gly Asp Lys Leu Cys
290 295 300

Thr Val Ala Thr Leu Arg Glu Thr Tyr Gly Glu Met Ala Asp Cys Cys
305 310 315 320

Ala Lys Gln Glu Pro Glu Arg Asn Glu Cys Phe Leu Gln His Lys Asp
325 330 335

Asp Asn Pro Asn Leu Pro Arg Leu Val Arg Pro Glu Val Asp Val Met
340 345 350

Cys Thr Ala Phe His Asp Asn Glu Glu Thr Phe Leu Lys Lys Tyr Leu
355 360 365

Tyr Glu Ile Ala Arg Arg His Pro Tyr Phe Tyr Ala Pro Glu Leu Leu
370 375 380

Phe Phe Ala Lys Arg Tyr Lys Ala Ala Phe Thr Glu Cys Cys Gln Ala
385 390 395 400

Ala Asp Lys Ala Ala Cys Leu Leu Pro Lys Leu Asp Glu Leu Arg Asp
405 410 415

Glu Gly Lys Ala Ser Ser Ala Lys Gln Arg Leu Lys Cys Ala Ser Leu
        420                 425                 430

Gln Lys Phe Gly Glu Arg Ala Phe Lys Ala Trp Ala Val Ala Arg Leu
        435                 440                 445

Ser Gln Arg Phe Pro Lys Ala Glu Phe Ala Glu Val Ser Lys Leu Val
        450                 455                 460

Thr Asp Leu Thr Lys Val His Thr Glu Cys Cys His Gly Asp Leu Leu
465                 470                 475                 480

Glu Cys Ala Asp Asp Arg Ala Asp Leu Ala Lys Tyr Ile Cys Glu Asn
                485                 490                 495

Gln Asp Ser Ile Ser Ser Lys Leu Lys Glu Cys Cys Glu Lys Pro Leu
        500                 505                 510

Leu Glu Lys Ser His Cys Ile Ala Glu Val Glu Asn Asp Glu Met Pro
        515                 520                 525

Ala Asp Leu Pro Ser Leu Ala Ala Asp Phe Val Glu Ser Lys Asp Val
        530                 535                 540

Cys Lys Asn Tyr Ala Glu Ala Lys Asp Val Phe Leu Gly Met Phe Leu
545                 550                 555                 560

Tyr Glu Tyr Ala Arg Arg His Pro Asp Tyr Ser Val Val Leu Leu Leu
                565                 570                 575

Arg Leu Ala Lys Thr Tyr Glu Thr Thr Leu Glu Lys Cys Cys Ala Ala
                580                 585                 590

Ala Asp Pro His Glu Cys Tyr Ala Lys Val Phe Asp Glu Phe Lys Pro
        595                 600                 605

Leu Val Glu Glu Pro Gln Asn Leu Ile Lys Gln Asn Cys Glu Leu Phe
        610                 615                 620

Glu Gln Leu Gly Glu Tyr Lys Phe Gln Asn Ala Leu Leu Val Arg Tyr
625                 630                 635                 640

Thr Lys Lys Val Pro Gln Val Ser Thr Pro Thr Leu Val Glu Val Ser
                645                 650                 655

Arg Asn Leu Gly Lys Val Gly Ser Lys Cys Cys Lys His Pro Glu Ala
                660                 665                 670

Lys Arg Met Pro Cys Ala Glu Asp Tyr Leu Ser Val Val Leu Asn Gln
        675             680             685

Leu Cys Val Leu His Glu Lys Thr Pro Val Ser Asp Arg Val Thr Lys
        690             695             700

Cys Cys Thr Glu Ser Leu Val Asn Arg Arg Pro Cys Phe Ser Ala Leu
705             710             715             720

Glu Val Asp Glu Thr Tyr Val Pro Lys Glu Phe Asn Ala Glu Thr Phe
            725             730             735

Thr Phe His Ala Asp Ile Cys Thr Leu Ser Glu Lys Glu Arg Gln Ile
        740             745             750

Lys Lys Gln Thr Ala Leu Val Glu Leu Val Lys His Lys Pro Lys Ala
        755             760             765

Thr Lys Glu Gln Leu Lys Ala Val Met Asp Asp Phe Ala Ala Phe Val
    770             775             780

Glu Lys Cys Cys Lys Ala Asp Asp Lys Glu Thr Cys Phe Ala Glu Glu
785             790             795             800

Gly Lys Lys Leu Val Ala Ala Ser Gln Ala Ala Leu Gly Leu Gly Gly
            805             810             815

Gly Gly Ser Gly Gly Gly Gly Ser Gly Gly Gly Gly Ser Asp Tyr Lys
        820             825             830

Asp Asp Asp Asp Lys
        835

<210> 16
<211> 711
<212> PRT
<213> Artificial

<220>
<223> LRRD2-10

<400> 16

Met Ala Arg Pro Leu Cys Thr Leu Leu Leu Leu Met Ala Thr Leu Ala
1               5               10              15

Gly Ala Leu Ala Leu Thr Ser Leu Val Leu Tyr Gly Asn Lys Ile Thr
            20              25              30

```
Glu Ile Ala Lys Gly Leu Phe Asp Gly Leu Val Ser Leu Gln Leu Leu
        35              40              45

Leu Leu Asn Ala Asn Lys Ile Asn Cys Leu Arg Val Asn Thr Phe Gln
        50              55              60

Asp Leu Gln Asn Leu Asn Leu Leu Ser Leu Tyr Asp Asn Lys Leu Gln
65              70              75              80

Thr Ile Ser Lys Gly Leu Phe Ala Gly Gly Gly Ser Gly Gly Gly
            85              90              95

Gly Ser Gly Gly Gly Gly Ser Asp Ala His Lys Ser Glu Val Ala His
            100             105             110

Arg Phe Lys Asp Leu Gly Glu Glu Asn Phe Lys Ala Leu Val Leu Ile
            115             120             125

Ala Phe Ala Gln Tyr Leu Gln Gln Cys Pro Phe Glu Asp His Val Lys
            130             135             140

Leu Val Asn Glu Val Thr Glu Phe Ala Lys Thr Cys Val Ala Asp Glu
145             150             155             160

Ser Ala Glu Asn Cys Asp Lys Ser Leu His Thr Leu Phe Gly Asp Lys
            165             170             175

Leu Cys Thr Val Ala Thr Leu Arg Glu Thr Tyr Gly Glu Met Ala Asp
            180             185             190

Cys Cys Ala Lys Gln Glu Pro Glu Arg Asn Glu Cys Phe Leu Gln His
            195             200             205

Lys Asp Asp Asn Pro Asn Leu Pro Arg Leu Val Arg Pro Glu Val Asp
            210             215             220

Val Met Cys Thr Ala Phe His Asp Asn Glu Glu Thr Phe Leu Lys Lys
225             230             235             240

Tyr Leu Tyr Glu Ile Ala Arg Arg His Pro Tyr Phe Tyr Ala Pro Glu
            245             250             255

Leu Leu Phe Phe Ala Lys Arg Tyr Lys Ala Ala Phe Thr Glu Cys Cys
            260             265             270

Gln Ala Ala Asp Lys Ala Ala Cys Leu Leu Pro Lys Leu Asp Glu Leu
            275             280             285
```

```
Arg Asp Glu Gly Lys Ala Ser Ser Ala Lys Gln Arg Leu Lys Cys Ala
    290                 295                 300

Ser Leu Gln Lys Phe Gly Glu Arg Ala Phe Lys Ala Trp Ala Val Ala
305                 310                 315                 320

Arg Leu Ser Gln Arg Phe Pro Lys Ala Glu Phe Ala Glu Val Ser Lys
                325                 330                 335

Leu Val Thr Asp Leu Thr Lys Val His Thr Glu Cys Cys His Gly Asp
            340                 345                 350

Leu Leu Glu Cys Ala Asp Asp Arg Ala Asp Leu Ala Lys Tyr Ile Cys
        355                 360                 365

Glu Asn Gln Asp Ser Ile Ser Ser Lys Leu Lys Glu Cys Cys Glu Lys
    370                 375                 380

Pro Leu Leu Glu Lys Ser His Cys Ile Ala Glu Val Glu Asn Asp Glu
385                 390                 395                 400

Met Pro Ala Asp Leu Pro Ser Leu Ala Ala Asp Phe Val Glu Ser Lys
            405                 410                 415

Asp Val Cys Lys Asn Tyr Ala Glu Ala Lys Asp Val Phe Leu Gly Met
        420                 425                 430

Phe Leu Tyr Glu Tyr Ala Arg Arg His Pro Asp Tyr Ser Val Val Leu
        435                 440                 445

Leu Leu Arg Leu Ala Lys Thr Tyr Glu Thr Thr Leu Glu Lys Cys Cys
    450                 455                 460

Ala Ala Ala Asp Pro His Glu Cys Tyr Ala Lys Val Phe Asp Glu Phe
465                 470                 475                 480

Lys Pro Leu Val Glu Glu Pro Gln Asn Leu Ile Lys Gln Asn Cys Glu
                485                 490                 495

Leu Phe Glu Gln Leu Gly Glu Tyr Lys Phe Gln Asn Ala Leu Leu Val
            500                 505                 510

Arg Tyr Thr Lys Lys Val Pro Gln Val Ser Thr Pro Thr Leu Val Glu
        515                 520                 525

Val Ser Arg Asn Leu Gly Lys Val Gly Ser Lys Cys Cys Lys His Pro
```

```
                530                      535                         540


        Glu Ala Lys Arg Met Pro Cys Ala Glu Asp Tyr Leu Ser Val Val Leu
        545             550             555                     560


        Asn Gln Leu Cys Val Leu His Glu Lys Thr Pro Val Ser Asp Arg Val
                    565             570                 575


        Thr Lys Cys Cys Thr Glu Ser Leu Val Asn Arg Arg Pro Cys Phe Ser
                    580             585                 590


        Ala Leu Glu Val Asp Glu Thr Tyr Val Pro Lys Glu Phe Asn Ala Glu
                    595             600                 605


        Thr Phe Thr Phe His Ala Asp Ile Cys Thr Leu Ser Glu Lys Glu Arg
            610             615                 620


        Gln Ile Lys Lys Gln Thr Ala Leu Val Glu Leu Val Lys His Lys Pro
        625             630             635                     640


        Lys Ala Thr Lys Glu Gln Leu Lys Ala Val Met Asp Asp Phe Ala Ala
                    645             650                 655


        Phe Val Glu Lys Cys Cys Lys Ala Asp Asp Lys Glu Thr Cys Phe Ala
                    660             665                 670


        Glu Glu Gly Lys Lys Leu Val Ala Ala Ser Gln Ala Ala Leu Gly Leu
                    675             680                 685


        Gly Gly Gly Gly Ser Gly Gly Gly Gly Ser Gly Gly Gly Gly Ser Asp
                    690             695                 700


        Tyr Lys Asp Asp Asp Asp Lys
        705                 710


        <210>   17
        <211>   773
        <212>   PRT
        <213>   Artificial

        <220>
        <223>   LRRD2-11

        <400>   17

        Met Ala Arg Pro Leu Cys Thr Leu Leu Leu Leu Met Ala Thr Leu Ala
        1               5                   10                  15


        Gly Ala Leu Ala Ile Val Glu Ile Arg Leu Glu Gln Asn Ser Ile Lys
                    20              25                  30
```

```
Ala Ile Pro Ala Gly Ala Phe Thr Gln Tyr Lys Lys Leu Lys Arg Ile
    35                  40              45

Asp Ile Ser Lys Asn Gln Ile Ser Asp Ile Ala Pro Asp Ala Phe Gln
    50                  55              60

Gly Leu Lys Ser Leu Thr Ser Leu Val Leu Tyr Gly Asn Lys Ile Thr
65                  70              75              80

Glu Ile Ala Lys Gly Leu Phe Asp Gly Leu Val Ser Leu Gln Leu Leu
            85                  90                  95

Leu Leu Asn Ala Asn Lys Ile Asn Cys Leu Arg Val Asn Thr Phe Gln
            100                 105                 110

Asp Leu Gln Asn Leu Asn Leu Leu Ser Leu Tyr Asp Asn Lys Leu Gln
        115                 120                 125

Thr Ile Ser Lys Gly Leu Phe Ala Pro Leu Gln Ser Ile Gln Thr Leu
    130                 135                 140

His Leu Ala Gln Asn Pro Gly Gly Gly Gly Ser Gly Gly Gly Gly Ser
145                 150                 155                 160

Gly Gly Gly Gly Ser Asp Ala His Lys Ser Glu Val Ala His Arg Phe
                165                 170                 175

Lys Asp Leu Gly Glu Glu Asn Phe Lys Ala Leu Val Leu Ile Ala Phe
            180                 185                 190

Ala Gln Tyr Leu Gln Gln Cys Pro Phe Glu Asp His Val Lys Leu Val
        195                 200                 205

Asn Glu Val Thr Glu Phe Ala Lys Thr Cys Val Ala Asp Glu Ser Ala
    210                 215                 220

Glu Asn Cys Asp Lys Ser Leu His Thr Leu Phe Gly Asp Lys Leu Cys
225                 230                 235                 240

Thr Val Ala Thr Leu Arg Glu Thr Tyr Gly Glu Met Ala Asp Cys Cys
            245                 250                 255

Ala Lys Gln Glu Pro Glu Arg Asn Glu Cys Phe Leu Gln His Lys Asp
        260                 265                 270

Asp Asn Pro Asn Leu Pro Arg Leu Val Arg Pro Glu Val Asp Val Met
```

275                            280                          285

Cys Thr Ala Phe His Asp Asn Glu Glu Thr Phe Leu Lys Lys Tyr Leu
    290               295               300

Tyr Glu Ile Ala Arg Arg His Pro Tyr Phe Tyr Ala Pro Glu Leu Leu
305             310              315               320

Phe Phe Ala Lys Arg Tyr Lys Ala Ala Phe Thr Glu Cys Cys Gln Ala
          325              330              335

Ala Asp Lys Ala Ala Cys Leu Leu Pro Lys Leu Asp Glu Leu Arg Asp
         340              345             350

Glu Gly Lys Ala Ser Ser Ala Lys Gln Arg Leu Lys Cys Ala Ser Leu
        355              360             365

Gln Lys Phe Gly Glu Arg Ala Phe Lys Ala Trp Ala Val Ala Arg Leu
       370             375             380

Ser Gln Arg Phe Pro Lys Ala Glu Phe Ala Glu Val Ser Lys Leu Val
385            390            395             400

Thr Asp Leu Thr Lys Val His Thr Glu Cys Cys His Gly Asp Leu Leu
          405             410             415

Glu Cys Ala Asp Asp Arg Ala Asp Leu Ala Lys Tyr Ile Cys Glu Asn
         420            425             430

Gln Asp Ser Ile Ser Ser Lys Leu Lys Glu Cys Cys Glu Lys Pro Leu
         435            440            445

Leu Glu Lys Ser His Cys Ile Ala Glu Val Glu Asn Asp Glu Met Pro
        450             455            460

Ala Asp Leu Pro Ser Leu Ala Ala Asp Phe Val Glu Ser Lys Asp Val
465           470           475             480

Cys Lys Asn Tyr Ala Glu Ala Lys Asp Val Phe Leu Gly Met Phe Leu
         485           490             495

Tyr Glu Tyr Ala Arg Arg His Pro Asp Tyr Ser Val Val Leu Leu Leu
        500            505            510

Arg Leu Ala Lys Thr Tyr Glu Thr Thr Leu Glu Lys Cys Cys Ala Ala
        515            520            525

```
Ala Asp Pro His Glu Cys Tyr Ala Lys Val Phe Asp Glu Phe Lys Pro
    530                 535                 540

Leu Val Glu Glu Pro Gln Asn Leu Ile Lys Gln Asn Cys Glu Leu Phe
545                 550                 555                 560

Glu Gln Leu Gly Glu Tyr Lys Phe Gln Asn Ala Leu Leu Val Arg Tyr
                565                 570                 575

Thr Lys Lys Val Pro Gln Val Ser Thr Pro Thr Leu Val Glu Val Ser
            580                 585                 590

Arg Asn Leu Gly Lys Val Gly Ser Lys Cys Cys Lys His Pro Glu Ala
        595                 600                 605

Lys Arg Met Pro Cys Ala Glu Asp Tyr Leu Ser Val Val Leu Asn Gln
    610                 615                 620

Leu Cys Val Leu His Glu Lys Thr Pro Val Ser Asp Arg Val Thr Lys
625                 630                 635                 640

Cys Cys Thr Glu Ser Leu Val Asn Arg Arg Pro Cys Phe Ser Ala Leu
                645                 650                 655

Glu Val Asp Glu Thr Tyr Val Pro Lys Glu Phe Asn Ala Glu Thr Phe
            660                 665                 670

Thr Phe His Ala Asp Ile Cys Thr Leu Ser Glu Lys Glu Arg Gln Ile
        675                 680                 685

Lys Lys Gln Thr Ala Leu Val Glu Leu Val Lys His Lys Pro Lys Ala
    690                 695                 700

Thr Lys Glu Gln Leu Lys Ala Val Met Asp Asp Phe Ala Ala Phe Val
705                 710                 715                 720

Glu Lys Cys Cys Lys Ala Asp Asp Lys Glu Thr Cys Phe Ala Glu Glu
                725                 730                 735

Gly Lys Lys Leu Val Ala Ala Ser Gln Ala Ala Leu Gly Leu Gly Gly
            740                 745                 750

Gly Gly Ser Gly Gly Gly Gly Ser Gly Gly Gly Gly Ser Asp Tyr Lys
        755                 760                 765

Asp Asp Asp Asp Lys
        770
```

```
<210>  18
<211>  852
<212>  PRT
<213>  Artificial

<220>
<223>  LRRD2-12

<400>  18
```

Met Ala Arg Pro Leu Cys Thr Leu Leu Leu Leu Met Ala Thr Leu Ala
1               5                   10                  15

Gly Ala Leu Ala Ile Ser Cys Pro Ser Pro Cys Thr Cys Ser Asn Asn
            20                  25                  30

Ile Val Asp Cys Arg Gly Lys Gly Leu Met Glu Ile Pro Ala Asn Leu
            35                  40                  45

Pro Glu Gly Ile Val Glu Ile Arg Leu Glu Gln Asn Ser Ile Lys Ala
        50                  55                  60

Ile Pro Ala Gly Ala Phe Thr Gln Tyr Lys Lys Leu Lys Arg Ile Asp
65                  70                  75                  80

Ile Ser Lys Asn Gln Ile Ser Asp Ile Ala Pro Asp Ala Phe Gln Gly
                85                  90                  95

Leu Lys Ser Leu Thr Ser Leu Val Leu Tyr Gly Asn Lys Ile Thr Glu
            100                 105                 110

Ile Ala Lys Gly Leu Phe Asp Gly Leu Val Ser Leu Gln Leu Leu Leu
            115                 120                 125

Leu Asn Ala Asn Lys Ile Asn Cys Leu Arg Val Asn Thr Phe Gln Asp
        130                 135                 140

Leu Gln Asn Leu Asn Leu Leu Ser Leu Tyr Asp Asn Lys Leu Gln Thr
145                 150                 155                 160

Ile Ser Lys Gly Leu Phe Ala Pro Leu Gln Ser Ile Gln Thr Leu His
            165                 170                 175

Leu Ala Gln Asn Pro Phe Val Cys Asp Cys His Leu Lys Trp Leu Ala
            180                 185                 190

Asp Tyr Leu Gln Asp Asn Pro Ile Glu Thr Ser Gly Ala Arg Cys Ser
            195                 200                 205

```
Ser Pro Arg Arg Leu Ala Asn Lys Arg Ile Ser Gln Ile Lys Ser Lys
    210             215             220

Lys Phe Arg Cys Ser Gly Gly Gly Ser Gly Gly Gly Ser Gly
    225             230             235             240

Gly Gly Gly Ser Asp Ala His Lys Ser Glu Val Ala His Arg Phe Lys
            245             250             255

Asp Leu Gly Glu Glu Asn Phe Lys Ala Leu Val Leu Ile Ala Phe Ala
        260             265             270

Gln Tyr Leu Gln Gln Cys Pro Phe Glu Asp His Val Lys Leu Val Asn
    275             280             285

Glu Val Thr Glu Phe Ala Lys Thr Cys Val Ala Asp Glu Ser Ala Glu
    290             295             300

Asn Cys Asp Lys Ser Leu His Thr Leu Phe Gly Asp Lys Leu Cys Thr
305             310             315             320

Val Ala Thr Leu Arg Glu Thr Tyr Gly Glu Met Ala Asp Cys Cys Ala
            325             330             335

Lys Gln Glu Pro Glu Arg Asn Glu Cys Phe Leu Gln His Lys Asp Asp
            340             345             350

Asn Pro Asn Leu Pro Arg Leu Val Arg Pro Glu Val Asp Val Met Cys
        355             360             365

Thr Ala Phe His Asp Asn Glu Glu Thr Phe Leu Lys Lys Tyr Leu Tyr
    370             375             380

Glu Ile Ala Arg Arg His Pro Tyr Phe Tyr Ala Pro Glu Leu Leu Phe
385             390             395             400

Phe Ala Lys Arg Tyr Lys Ala Ala Phe Thr Glu Cys Cys Gln Ala Ala
            405             410             415

Asp Lys Ala Ala Cys Leu Leu Pro Lys Leu Asp Glu Leu Arg Asp Glu
            420             425             430

Gly Lys Ala Ser Ser Ala Lys Gln Arg Leu Lys Cys Ala Ser Leu Gln
    435             440             445

Lys Phe Gly Glu Arg Ala Phe Lys Ala Trp Ala Val Ala Arg Leu Ser
    450             455             460
```

70

Gln Arg Phe Pro Lys Ala Glu Phe Ala Glu Val Ser Lys Leu Val Thr
465                 470                 475                 480

Asp Leu Thr Lys Val His Thr Glu Cys Cys His Gly Asp Leu Leu Glu
                485                 490                 495

Cys Ala Asp Asp Arg Ala Asp Leu Ala Lys Tyr Ile Cys Glu Asn Gln
            500                 505                 510

Asp Ser Ile Ser Ser Lys Leu Lys Glu Cys Cys Glu Lys Pro Leu Leu
        515                 520                 525

Glu Lys Ser His Cys Ile Ala Glu Val Glu Asn Asp Glu Met Pro Ala
    530                 535                 540

Asp Leu Pro Ser Leu Ala Ala Asp Phe Val Glu Ser Lys Asp Val Cys
545                 550                 555                 560

Lys Asn Tyr Ala Glu Ala Lys Asp Val Phe Leu Gly Met Phe Leu Tyr
            565                 570                 575

Glu Tyr Ala Arg Arg His Pro Asp Tyr Ser Val Val Leu Leu Leu Arg
            580                 585                 590

Leu Ala Lys Thr Tyr Glu Thr Thr Leu Glu Lys Cys Cys Ala Ala Ala
        595                 600                 605

Asp Pro His Glu Cys Tyr Ala Lys Val Phe Asp Glu Phe Lys Pro Leu
    610                 615                 620

Val Glu Glu Pro Gln Asn Leu Ile Lys Gln Asn Cys Glu Leu Phe Glu
625                 630                 635                 640

Gln Leu Gly Glu Tyr Lys Phe Gln Asn Ala Leu Leu Val Arg Tyr Thr
            645                 650                 655

Lys Lys Val Pro Gln Val Ser Thr Pro Thr Leu Val Glu Val Ser Arg
            660                 665                 670

Asn Leu Gly Lys Val Gly Ser Lys Cys Cys Lys His Pro Glu Ala Lys
        675                 680                 685

Arg Met Pro Cys Ala Glu Asp Tyr Leu Ser Val Val Leu Asn Gln Leu
    690                 695                 700

Cys Val Leu His Glu Lys Thr Pro Val Ser Asp Arg Val Thr Lys Cys
705                 710                 715                 720

71

```
Cys Thr Glu Ser Leu Val Asn Arg Arg Pro Cys Phe Ser Ala Leu Glu
            725                 730                 735

Val Asp Glu Thr Tyr Val Pro Lys Glu Phe Asn Ala Glu Thr Phe Thr
            740                 745                 750

Phe His Ala Asp Ile Cys Thr Leu Ser Glu Lys Glu Arg Gln Ile Lys
            755                 760                 765

Lys Gln Thr Ala Leu Val Glu Leu Val Lys His Lys Pro Lys Ala Thr
            770                 775                 780

Lys Glu Gln Leu Lys Ala Val Met Asp Asp Phe Ala Ala Phe Val Glu
    785                 790                 795                 800

Lys Cys Cys Lys Ala Asp Asp Lys Glu Thr Cys Phe Ala Glu Glu Gly
            805                 810                 815

Lys Lys Leu Val Ala Ala Ser Gln Ala Ala Leu Gly Leu Gly Gly Gly
            820                 825                 830

Gly Ser Gly Gly Gly Gly Ser Gly Gly Gly Gly Ser Asp Tyr Lys Asp
            835                 840                 845

Asp Asp Asp Lys
            850
```

**Description**

[Technical Field]

The present invention relates to a composition comprising albumin-bound Slit3 LRRD2 for prevention or treatment of a muscle disease, and more particularly provides a fusion protein comprising albumin-bound Slit3 LRRD2, a nucleic acid molecule encoding the fusion protein, a recombinant vector comprising the nucleic acid molecule, a transformant comprising the recombinant vector, a method for preparing a fusion protein using the transformant, a composition comprising the fusion protein for prevention or treatment of a muscle disease, and a composition comprising the fusion protein for improving the *in vivo* half-life of LRRD2 of the Slit 3 protein.

[Background Art]

Slit proteins are well-known proteins that regulate the movement of neurons and axons during the developmental process of the nervous system. It is known that a Slit protein can act with a Robo receptor to regulate physiological activity, and serves as a factor that regulates various intracellular processes in various tissues such as heart, lung, kidney, and breast tissues, and as it has been recently reported that Slit proteins play an important role in the regulation of growth, adhesion ability, and migration ability of cells, it was reported that Slit proteins can participate in the migration in the differentiation of cells and the occurrence and metastasis of cancer. Specifically, it was reported that Slit proteins and Robo proteins are expressed in the embryonic development stage of a vertebrate, and the expression of Slit3, Robo1 and Robo2 proteins is increased in the muscle tissues. The report mentioned that the expression of the Slit3 protein was increased in the myoblasts of hind leg muscle tissues of an embryo, but the protein might only participate in the migration ability and might not be associated with the differentiation of myoblasts.

The present inventors have revealed that LRRD2 of the Slit3 protein can be used for prevention or treatment of sarcopenia by promoting the differentiation of myoblasts to induce an increase in muscle mass (Korean Patent Application Laid-Open No. 10-2017-0138920). Since LRRD2 needs to be administered as an injection, patients need to visit the hospital, but LRRD2 has a very short in vivo half-life, so its administration cycle should be shortened in order to exhibit the medicinal effects thereof, and it is expected that a problem in that its efficacy is reduced due to the associated excessive use of the drug occurs.

Thus, the present inventors have developed an HSA-Slit3 LRRD2 fusion protein with improved efficacy by increasing the *in vivo* half-life of LRRD2, thereby completing the present invention.

[Disclosure]

[Technical Problem]

An object of the present invention is to provide a fusion protein for improving the *in vivo* half-life of LRRD2 of the Slit3 protein.

Another object of the present invention is to provide a nucleic acid molecule encoding the above-described fusion protein, a recombinant vector comprising the nucleic acid molecule, and a transformant comprising the recombinant vector.

Still another object of the present invention is to provide a method for preparing the above-described fusion protein.

Yet another object of the present invention is to provide a composition in which LRRD2 of the Slit3 protein has improved preventive or treatment efficacy for a muscle disease.

Yet another object of the present invention is to provide a composition in which LRRD2 of the Slit3 protein has an enhanced *in vivo* half-life.

[Technical Solution]

To achieve the above-described objects, the present invention provides a fusion protein in which albumin is bound to LRRD2 of the Slit3 protein.

According to a preferred exemplary embodiment of the present invention, the albumin may be human serum albumin.

According to another preferred exemplary embodiment of the present invention, in the fusion protein, the human serum albumin may be bound to the N-terminus of LRRD2 of the Slit3 protein.

According to still another preferred exemplary embodiment of the present invention, the human serum albumin may include an amino acid sequence of SEQ ID NO: 2.

According to yet another preferred exemplary embodiment of the present invention, the LRRD2 of the Slit3 protein may include an amino acid sequence of SEQ ID NO: 3.

According to yet another preferred exemplary embodiment of the present invention, a linker may be further included between the albumin and the LRRD2 of the Slit3 protein.

According to yet another preferred exemplary embodiment of the present invention, the linker may be (GGGGS)n (SEQ ID NO: 5), wherein n may be an integer from 1 to 10.

The present invention also provides a nucleic acid molecule encoding the above-described fusion protein.

The present invention also provides a recombinant vector comprising the above-described nucleic acid molecule and a transformant comprising the same.

The present invention also provides a method for preparing a fusion protein, the method comprising culturing the above-described transformant.

The present invention also provides a pharmaceutical composition comprising the above-described fusion protein for prevention or treatment of a muscle disease.

According to yet another preferred exemplary embodiment of the present invention, the pharmaceutical composition may be administered as an injection.

According to yet another preferred exemplary embodiment of the present invention, the muscle disease may be any one or more selected from the group consisting of atony, muscular atrophy, muscular dystrophy, cachexia, and sarcopenia.

The present invention also provides a composition comprising albumin-bound LRRD2 of the Slit3 protein for improving the *in vivo* half-life of LRRD2 of the Slit3 protein.

[Advantageous Effects]

Since the albumin-bound LRRD2 of the Slit3 protein exhibits the same cytological efficacy as albumin-unbound LRRD2 of the Slit3 protein and has a significantly increased *in vivo* half-life compared to albumin-unbound LRRD2 of the Slit3 protein, a bone-related disease can be more effectively prevented or treated.

[Description of Drawings]

FIG. 1 illustrates a composition of a fusion protein in which albumin of the present invention is bound to the N-terminus of Slit3 LRRD2 and an amino acid sequence thereof.

FIG. 2 illustrates the results of performing SDS-PAGE after isolating and purifying an SP cystatin S-HSA-Slit3LRRD2 fusion protein.

FIG. 3 graphically illustrates the receptor binding ability of various forms of HSA-Slit3 LRRD2 fusion proteins.

FIG. 4 illustrates the plasma concentration-time profiles of Slit3 LRRD2 after IV administration of Slit3 LRRD2 (●, "Slit3") and HSA-Slit3 LRRD2 (■, "HSA-Slit3 ") to fasted male ICR mice.

[Modes of the Invention]

As described above, LRRD2 of the Slit3 protein may be used for prevention or treatment of sarcopenia by promoting the differentiation of myoblasts to induce an increase in muscle mass, but LRRD2 has a very short *in vivo* half-life, so its administration cycle should be shortened in order to exhibit the medicinal effects thereof, and it is expected that a problem in that its efficacy is reduced due to the associated excessive use of the drug occurs

Thus, the present inventors have sought a solution to the above-described problem by enhancing the *in vivo* half-life of LRRD2 to develop an HSA-Slit3 LRRD2 fusion protein with improved efficacy. Since the albumin-bound LRRD2 of the Slit3 protein exhibits the same cytological efficacy as albumin-unbound LRRD2 of the Slit3 protein and has a significantly increased in vivo half-life compared to albumin-unbound LRRD2 of the Slit3 protein, a muscle-related disease can be more effectively prevented or treated.

Hereinafter, the present invention will be described in more detail.

The present invention provides a fusion protein in which albumin is bound to LRRD2 of the Slit3 protein.

In the fusion protein of the present invention, the "LRRD2 of the Slit3 protein" refers to a second leucine-rich repeat domain (LRRD2) in the Slit3 protein. Through previous studies, the present inventors confirmed that the Slit3 protein or LRRD2 in this protein binds to the Robo1 or Robo2 receptor to release the β-catenin binding to the M-cadherin of myoblasts via the Slit-Robo system, thereby promoting the formation of muscles by activating the β-catenin and increasing the expression of myogenin to induce the differentiation of myoblasts. As used herein, the term "Slit3 LRRD2" refers to "LRRD2 of the Slit3 protein" and may be used interchangeably.

In the fusion protein of the present invention, the albumin may be human serum albumin, rhesus serum albumin (RhSA), cynomolgus monkey serum albumin (CySA), or murine serum albumin (MuSA), and preferably human serum albumin. The Slit3 LRRD2 has an in vivo half-life of Slit3 LRRD2 in the presence of human serum albumin that is at least 10-fold longer than that of Slit3 LRRD2 in the absence of human serum albumin. In a specific exemplary embodiment of the present invention, the serum half-life of Slit3 LRRD2 in the presence of human serum albumin is 14-fold longer than that of Slit3 LRRD2 in the absence of human serum albumin.

The fusion protein according to the present invention may be bound in the order of the human serum albumin and Slit3 LRRD2, or vice versa. Preferably, the human serum albumin and Slit3 LRRD2 are bound in this order. For example, when the human serum albumin binds to the N-terminus of Slit3 LRRD2, Slit3 LRRD2 has the best *in vivo* half-life and therapeutic efficacy for muscle-related diseases, and when the human serum albumin binds to the C-terminus thereof, it is possible to exhibit an effective efficacy even though there may be a difference in degree.

In the fusion composition of the present invention, as the human serum albumin, a full-length amino acid sequence consisting of 609 amino acids or a fragment comprising a partial amino acid sequence thereof may be used. The full-length amino acid sequence of the human serum albumin is disclosed in the NCBI GenBank: AAA98797.1, and in an exemplary embodiment of the present invention, the form of a fragment consisting of the 25th to 609th amino acids (585 amino acids) from the full-length human serum albumin consisting of 609 amino acids was used. In the fusion protein of the present invention, the human serum albumin consists of the following SEQ ID NO: 2:

DAHKSEVAHRFKDLGEENFKALVLIAFAQYLQQCPFEDHVKLVNEVT

EFAKTCVADESAENCDKSLHTLFGDKLCTVATLRETYGEMADCCAKQEPER

NECFLQHKDDNPNLPRLVRPEVDVMCTAFHDNEETFLKKYLYEIARRHPYF

YAPELLFFAKRYKAAFTECCQAADKAACLLPKLDELRDEGKASSAKQRLKC

ASLQKFGERAFKAWAVARLSQRFPKAEFAEVSKLVTDLTKVHTECCHGDLL

ECADDRADLAKYICENQDSISSKLKECCEKPLLEKSHCIAEVENDEMPADLPS

LAADFVESKDVCKNYAEAKDVFLGMFLYEYARRHPDYSVVLLLRLAKTYE

TTLEKCCAAADPHECYAKVFDEFKPLVEEPQNLIKQNCELFEQLGEYKFQNA

LLVRYTKKVPQVSTPTLVEVSRNLGKVGSKCCKHPEAKRMPCAEDYLSVVL

NQLCVLHEKTPVSDRVTKCCTESLVNRRPCFSALEVDETYVPKEFNAETFTF

HADICTLSEKERQIKKQTALVELVKHKPKATKEQLKAVMDDFAAFVEKCCK

ADDKETCFAEEGKKLVAASQAALGL (SEQ ID NO: 2).

In the fusion protein of the present invention, the Slit3 LRRD2 is human-derived, and a full-length amino acid sequence of LRRD2 in the Slit3 protein consisting of 1523 amino acids or a fragment comprising a partial amino acid sequence thereof may be used. The full-length amino acid sequence of the Slit3 protein is disclosed in the NCBI GenBank: AAQ89243.1, and in an exemplary embodiment of the present invention, as Slit3 LRRD2, the form of a fragment consisting of the 278th to 486th amino acids (209 amino acids) from the full-length Slit3 protein consisting of 1523 amino acids was used. In the fusion protein of the present invention, Slit3 LRRD2 consists of an amino acid sequence of the following SEQ ID NO: 3:

ISCPSPCTCSNNIVDCRGKGLMEIPANLPEGIVEIRLEQNSIKAIPAGAF

TQYKKLKRIDISKNQISDIAPDAFQGLKSLTSLVLYGNKITEIAKGLFDGLVSL

QLLLLNANKINCLRVNTFQDLQNLNLLSLYDNKLQTISKGLFAPLQSIQTLHL

AQNPFVCDCHLKWLADYLQDNPIETSGARCSSPRRLANKRISQIKSKKFRCS

(SEQ ID NO: 3).

In the fusion protein of the present invention, "Slit3 LRRD2" may include a functional equivalent of the amino acid sequence of SEQ ID NO: 3.

The "functional equivalent" has a sequence homology of at least 70% or more, preferably 80% or more, more preferably 90% or more, and even more preferably 95% or more with the amino acid sequences of SEQ ID NOS: 1 to 4 of the present invention by the addition, substitution, or deletion of amino acids of a protein or peptide, and refers to a protein or peptide exhibiting physiological activity substantially equivalent to that of a protein or peptide consisting of amino acid sequences of SEQ ID NOS: 1 to 4.

Specifically, for the fusion protein, not only a protein or peptide having a wild-type amino acid sequence thereof, but also an amino acid sequence variant thereof may also be included in the scope of the present invention. The amino acid sequence variant refers to a protein or peptide having a sequence different from a wild-type amino acid sequence of Slit3 LRRD2 by deletion, insertion, non-conservative or conservative substitution of one or more amino acid residues, or a combination thereof.

Amino acid exchanges possible in proteins and peptides that do not entirely change the activities of the molecules are known in the art (H. Neurath, R.L.Hill, The Proteins, Academic Press, New York, 1979). The most typically occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may also be modified by phosphorylation, sulfation, acetylation, glycosylation, methylation, farnesylation, or the like.

The Slit3 LRRD2 of the present invention, or variants thereof can be extracted from nature or synthesized (Merrifleld, J. Amer. Chem. Soc. 85:2149-2156, 1963) or prepared by a gene recombinant method based on a DNA sequence (Sambrook et al., Molecular Cloning, Cold Spring Harbour Laboratory Press, New York, USA, 2d Ed., 1989).

The Slit3 LRRD2 of the present invention may be subjected not only to albumin fusion, but also to fusion or PEGylation of an Fc protein of IgG, and the like in order to enhance the *in vivo* half-life thereof.

In the fusion protein of the present invention, a linker may be further included between albumin and LRRD2 of the Slit3 protein. A preferred linker type may be (GGGS)n (SEQ ID NO: 5), wherein n may be an integer from 1 to 10, and preferably n may be an integer from 1 to 5.

The present invention also provides a nucleic acid molecule encoding a fusion protein in the form of human serum albumin being linked to the N-terminus of Slit3 LRRD2, and additionally, cystatin S may be linked to the N-terminus of the human serum albumin.

In the nucleic acid molecule of the present invention, cystatin S is a signal peptide, a base sequence encoding the cystatin S is linked to the 5' terminus of a base sequence encoding a human serum albumin, and a base sequence encoding the human serum albumin may be linked to the 5' terminus of a base sequence encoding Slit3 LRRD2 to produce cystatin S-HSA-Slit3 LRRD

In the nucleic acid molecule of the present invention, the cystatin S is human-derived, and may be used as a base sequence encoding a full-length amino acid sequence consisting of 141 amino acids or a partial amino acid sequence thereof. The full-length amino acid sequence of cystatin S is disclosed in the NCBI GenBank: EAX10135.1, and in an exemplary embodiment of the present invention, in a full-length cystatin S consisting of 141 amino acids, a base sequence encoding a fragment consisting of the 1st to 20th amino acids was used. In the nucleic acid molecule of the present invention, cystatin S is a base sequence encoding an amino acid sequence of the following SEQ ID NO: 1. MARPLCTLLLLMATLAGALA (SEQ ID NO: 1).

The present invention also provides a recombinant vector comprising a base sequence encoding the fusion protein and a promoter functionally linked to the base sequence.

The term "functionally linked" means being functionally linked between a nucleic acid expression regulatory sequence (an array of a promoter, a signal sequence, or a transcriptional regulatory factor binding site) and a secondary base sequence, and the expression regulatory sequence affects the transcription and/or translation of nucleic acids corresponding to the secondary sequence.

The vector system of the present invention may be prepared by a method well-known in the art as described in the literature [Sambrook et al., Molecular Cloning, A Laboratory Manual, Cold Spring Harbor Laboratory Press(2001)].

In general, the vector may be prepared for cloning or expression purposes. Further, the vector may be prepared for use in eukaryotic or prokaryotic host cells. For example, when a vector is prepared for expression in prokaryotic cells, the vector includes a strong promoter for initiating transcription (for example, a pLλ promoter, a trp promoter, a lac promoter, a tac promoter and a T7 promoter), a ribosome binding site or a sequence for initiating translation and stopping transcription/translation. In particular, when *E. coli* is used as a host cell, for biosynthesis of tryptophan in *E. coli,* a promoter and an operator in an operon (Yanofsky, C., J. Bacteriol., 158: 1018-1024 (1984)) and a left directional promoter of phage λ (a pLλ promoter, Herskowitz, I. and Hagen, D., Ann. Rev. Genet., 14: 399-445 (1980)) may be used as regulatory sequences. When Bacillus is used as a host cell, a promoter for a gene encoding a toxin protein of *Bacillus thuringiensis* (Appl. Environ. Microbiol. 64: 3932-3938 (1998); and Mol. Gen. Genet. 250: 734-741 (1996)) or another operable promoter in Bacillus may be used as a regulatory sequence.

Numerous typical vectors available for prokaryotic cells are well known to those skilled in the art, and selection of a suitable vector is a matter of choice. A typical vector used in the present invention includes pSC101, pGV1106, pACYC177, ColE1, pKT230, pME290, pBR322, pUC8 / 9, pUC6, pBD9, pHC79, pIJ61, pLAFR1, pHV14, pGEX series, pET series, pUC19, λgt • 4 λB, λ-charon, λΔz1 and M13 and is not necessarily limited thereto.

For example, when a vector is prepared for a eukaryotic host cell, among them, a promoter derived from the genome of an animal cell or a mammalian cell (for example, a metallothionein promoter) or a mammalian virus (for example, an adenovirus late promoter; a vaccinia 7.5 K promoter, an SV40 promoter, a cytomegalovirus promoter and a tk promoter of HSV) may be used. The vector generally includes a polyadenylation site of a transcript. Examples of a commercially available virus-based vector include pcDNA 3 (Invitrogen; including a cytomegalovirus promoter and a polyadenylation signal), pSI (Promega; including an SV 40 promoter and a polyadenylation signal), pCI (Promega; including a cytomegalovirus promoter and a polyadenylation signal), and pREP7 (Invitrogen; including a RSV promoter and an SV 40 polyadenylation signal).

When a vector is prepared for yeast, the promoters of the genes for phosphoglycerate kinase, glyceraldehyde-3-phosphate dehydrogenase, lactase, enolase and alcohol dehydrolase may be used as regulatory sequences.

When an expression vector is prepared for plant cells, various plant functional promoters known in the art may be used, and include a cauliflower mosaic virus (CaMV) 35S promoter, the pigwarm mosaic virus 35S promoter, a sugarcane bacilliform virus promoter, a Comerina yellow mottle virus promoter, a light-inducible promoter from subunit of ribulose-1,5-bis-phosphate carboxylase (ssRUBISCO), a rice cytosolic triosephosphate isomerase (TPI) promoter, an adenine phosphoribosyltransferase (APRT) promoter from Arabidopsis, a rice actin 1 gene promoter and mannopine synthase and octopine synthase promoters.

In addition, the recombinant vector of the present invention includes a base sequence capable of easily separating an expressed fusion protein, and includes, but is not limited to, glutathione S-transferase (Pharmacia, USA), a maltose binding protein (NEB, USA), FLAG (IBI, USA) and 6X His (hexahistidine; Qiagen, USA). Fusion proteins expressed by such an additional sequence may be separated by affinity chromatography in a rapid and convenient manner.

In a specific exemplary embodiment of the present invention, a FLAG sequence was used, and a FLAG protein includes an amino acid sequence of SEQ ID NO: 4. The amino acid sequence of SEQ ID NO: 4 is as follows: DYKDDDDK (SEQ ID NO: 4).

According to another exemplary embodiment of the present invention, the fusion protein is separated by affinity chromatography. For example, in the case of glutathione S-transferase, an elution buffer including glutathione is used, and when 6X His is used to separate a foreign protein, a Ni-NTA His-binding resin (Novagen, USA) is used.

The expression vector of the present invention preferably includes one or more markers capable of selecting a transformed host, and examples thereof include genes resistant to antibiotics such as ampicillin, gentamicin, chloramphenicol, streptomycin, kanamycin, neomycin, geneticin and tetracycline, URA3 genes, and genes having resistance to other toxic compounds such as metal ions.

The present invention also provides a transformant comprising the recombinant vector described above.

A host useful for preparing a transformant is well-known in the art. For example, it is possible to use a prokaryotic host, *E. coli* JM109, *E. coli* BL21, *E. coli* RR1, *E. coli* LE392, *E. coli* B, *E. coli* X 1776, *E. coli* W3110, *Bacillus subtilis, Bacillus thuringiensis, Salmonella typhimurium, Serratia marcescens* and various Pseudomonas species, Corynebacterium and Streptomyces.

In eukaryotic cells, yeast (*Saccharomyces cerevisiae*), insect cells, human cells (for example, CHO, W138, BHK, COS-7, 293, HepG2, 3T3, RIN and MDCK cell lines) and plant somatic cells may be used.

Transformation of host cells may be performed by a number of methods known in the art. For example, when prokaryotic cells are used as host cells, a CaC12 method, a Hanson method (Cohen, S.N. et al., Proc. Natl. Acac. Sci. USA, 9:2110-2114(1973); and Hanahan, D., J. Mol. Biol., 166:557-580(1983)) and electrophoresis may be used for transformation. Further, when eukaryotic cells are used as host cells, microinjection, calcium phosphate precipitation, electric shock, liposome-mediated phenotypic infection, DEAE-dextran treatment, and particle bombardment may be used for transformation. In addition, when plant cells are used as host cells, Agrobacterium-mediated transformation is the most preferable method, the reason for which is enabling a bypass required for redifferentiation of adjacent plants from protoplasts.

The present invention also provides a method for producing the HSA-Slit3 LRRD2 fusion protein. The method includes (a) culturing the transformant described above under conditions for expression; and (b) obtaining a produced fusion protein.

A transformant for preparing the fusion protein of the present invention may be cultured using a suitable medium and culture conditions known in the art. These culturing processes may be easily adjusted and used by those skilled in the art according to the selected strain. Cell culture is classified into suspension culture and adherent culture depending on the cell growth method, and into a batch culture type, a fed-batch type and a continuous culture type depending on the culture method. Media used for culturing need to appropriately meet the requirements of a particular strain.

A medium used for culturing animal cells contains various carbon sources, nitrogen sources, and trace element components. Examples of a carbon source which may be used include carbohydrates such as glucose, sucrose, lactose, fructose, maltose, starch and cellulose, fats such as soybean oil, sunflower oil, hemp oil and coconut oil, fatty acids such as palmitic acid, stearic acid and linoleic acid, alcohols such as glycerol and ethanol, and organic acids such as acetic acid. These carbon sources may be used either alone or in combination. Examples of a nitrogen source which may be used include organic nitrogen sources such as peptone, yeast extract, meat juice, malt extract, corn steep liquid (CSL) and soybean meal and inorganic nitrogen sources such as urea, ammonium sulfate, ammonium chloride, ammonium phosphate, ammonium carbonate and ammonium nitrate. These nitrogen sources may be used either alone or in combination. The medium may include potassium dihydrogen phosphate, dipotassium hydrogen phosphate and the corresponding sodium-containing salt as a phosphorus source. Furthermore, the medium may include a metal salt such as magnesium sulfate or iron sulfate. In addition to the above, amino acids, vitamins, an appropriate precursor, and the like may be included.

During the culturing, the pH of a culture may be adjusted by adding a compound such as ammonium hydroxide, potassium hydroxide, ammonia, phosphoric acid and sulfuric acid to the cultured product. Further, the formation of bubbles may be suppressed using a defoaming agent such as fatty acid polyglycol ester. In addition, in order to maintain the aerobic state of the culture, oxygen or an oxygen-containing gas (for example, air) is injected into the culture. The temperature of the culture is usually 20°C to 45°C, preferably 25°C to 40°C.

In the method for producing the HSA-Slit3 LRRD2 fusion protein of the present invention, the obtaining of the produced fusion protein in step (b) may be performed in order to obtain a fusion protein in a separated form. For example, when a fusion protein is expressed by a large volume of transformed bacteria, the fusion protein is generally expressed after promoter induction, but expression continues, and the protein forms an insoluble precipitate (that is, an inclusion body). There are several suitable protocols for the separation of the inclusion body. The fused protein that formed the inclusion body may be reformed through dilution or dialysis using a suitable buffer. And then, the fusion protein may be purified using a basic method known in the art including solubility fragmentation by the use of ammonium sulfate, size differential filtration (ultrafiltration) and column chromatography (depending on size, net surface charge, hydrophobicity or affinity).

The fusion protein of the present invention may be expressed in plants. Plant cells may be transformed by a typical method known in the art, and may be transformed by electric shock, particle bombardment and Agrobacterium-induced transformation. Among them, Agrobacterium-induced transformation is most preferred. Agrobacterium-induced transformation may generally be performed using leaf slices and other tissues such as cotyledon and hypocotyl.

Transformed cells may be selected by exposing a transformed culture to a selected preparation such as a metabolic inhibitor, an antibiotic and an herbicide. When cells are transformed and stably express a marker gene having resistance to a selected gene, the cells grow and differentiate during the culturing. For example, a marker includes, but is not limited to, a glycophospate resistant gene and a neomycin phosphotransferase (nptII) system. The development or redifferentiation of plants from plant protoplasts or various foreign materials is well known in the art. The resulting transformed root shoots are seeded in a suitable plant growth medium. Development or redifferentiation of plants including foreign genes induced by Agrobacterium may be performed by methods known in the art.

The present invention provides a pharmaceutical composition comprising albumin-bound LRRD2 of the Slit3 protein for prevention or treatment of a muscle disease.

The pharmaceutical composition of the present invention may be in the form of various oral or parenteral formulations. When the pharmaceutical composition is formulated, the composition may be prepared by using a buffer (for example, a saline solution or PBS), an antioxidant, a bacteriostatic agent, a chelating agent (for example, EDTA or glutathione), a filler, an extender, a binder, an adjuvant (for example, aluminum hydroxide), a suspension agent, a thickener, a wetting agent, a disintegrant, or a surfactant, a diluent or an excipient.

Examples of a solid preparation for oral administration include a tablet, a pill, a powder, granules, a capsule, and the like, and the solid preparation is prepared by mixing one or more compounds with one or more excipients, for example, starch (including corn starch, wheat starch, rice starch, potato starch, and the like), calcium carbonate, sucrose, lactose, dextrose, sorbitol, mannitol, xylitol, erythritol maltitol, cellulose, methyl cellulose, sodium carboxymethylcellulose and hydroxypropymethyl-cellulose, gelatin, or the like. For example, a tablet or a sugar tablet may be obtained by blending an active ingredient with a solid excipient, pulverizing the resulting blend, adding a suitable auxiliary agent thereto, and then processing the resulting mixture into a granular mixture.

Further, in addition to simple excipients, lubricants such as magnesium stearate and talc are also used. A liquid preparation for oral administration corresponds to a suspension agent, a liquid for internal use, an emulsion, a syrup, and the like, and the liquid preparation may include, in addition to water and liquid paraffin which are simple commonly used diluents, various excipients, for example, a wetting agent, a sweetener, an odorant, a preservative, and the like. In addition, in some cases, cross-linked polyvinyl pyrrolidone, agar, alginic acid, sodium alginate, or the like may be added as a disintegrant, and an anti-coagulant, a lubricant, a wetting agent, a flavoring agent, an emulsifier, an antiseptic, and the like may be additionally added.

Examples of a preparation for parenteral administration include an aqueous sterile solution, a non-aqueous solvent, a suspension solvent, an emulsion, a freeze-dried preparation, a suppository, or the like. As the non-aqueous solvent and the suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerol, gelatin, and the like.

The pharmaceutical composition of the present invention may be administered orally or parenterally, and, when administered parenterally, may be formulated in the form of a preparation for external application to the skin; an injection administered intraperitoneally, rectally, intravenously, muscularly, subcutaneously, or intracerebroventricularly, or via cervical intrathecal injection; a percutaneous administration agent; or a nasal inhaler according to a method known in the art.

The injection must be sterilized and protected from contamination of microorganisms such as bacteria and fungi. Examples of a suitable carrier for the injection may be, but are not limited to, a solvent or a dispersion medium including water, ethanol, polyols (for example, glycerol, propylene glycol, liquid polyethylene glycol, and the like), mixtures thereof, and/or vegetable oils. More preferably, as a suitable carrier, it is possible to use an isotonic solution such as Hank's solution, Ringer's solution, triethanolamine-containing phosphate buffered saline (PBS) or sterile water for injection, 10% ethanol, 40% propylene glycol, and 5% dextrose, and the like. To protect the injection from microbial contamination, various antimicrobial agents and antifungal agents such as a paraben, chlorobutanol, phenol, sorbic acid, and thimerosal may be additionally included. Furthermore, in most cases, the injection may additionally include an isotonic agent such as sugar or sodium chloride.

Examples of the percutaneous administration agent include a form such as an ointment, a cream, a lotion, a gel, a solution for external use, a paste, a liniment, and an aerosol. The transdermal administration as described above means that an effective amount of an active ingredient contained in a pharmaceutical composition is delivered into the skin via local administration thereof to the skin.

In the case of a preparation for inhalation, the fusion protein used according to the present invention may be conveniently delivered in the form of an aerosol spray from a pressurized pack or a nebulizer by using a suitable propellant, for example, dichlorofluoromethane, trichlorofluoromethane, dichlorotetrafluoroethane, carbon dioxide, or other suitable gases. In the case of the pressurized aerosol, a dosage unit may be determined by providing a valve for transferring a metered amount. For example, a gelatin capsule and a cartridge for use in an inhaler or insufflator may be formulated so as to contain a powder mixture of a compound and a suitable powder base such as lactose or starch. Formulations for parenteral administration are described in the document, which is a guidebook generally known in all pharmaceutical chemistry fields (Remington's Pharmaceutical Science, 15th Edition, 1975. Mack Publishing Company, Easton, Pennsylvania 18042, Chapter 87: Blaug, Seymour).

The pharmaceutical composition of the present invention is administered in a pharmaceutically effective amount. The term "pharmaceutically effective amount" as used herein refers to an amount sufficient to treat diseases at a reasonable benefit/risk ratio applicable to medical treatment, and an effective dosage level may be determined according to factors including type of diseases of patients, the severity of disease, the activity of drugs, sensitivity to drugs, administration time, administration routes, excretion rate, treatment periods, and simultaneously used drugs, and other factors well known in the medical field. The pharmaceutical composition of the present invention may be administered as an individual therapeutic agent or in combination with other therapeutic agents, may be administered sequentially or simultaneously with therapeutic agents in the related art, and may be administered in a single dose or multiple doses. That is, the total effective amount of the composition of the present invention may be administered to a patient in a single dose or may be administered by a fractionated treatment protocol, in which multiple doses are administered over a long period of time. It is important to administer the composition in a minimum amount that can obtain the maximum effect without any side effects, in consideration of all the aforementioned factors, and this amount may be easily determined by those skilled in the art.

A dosage of the pharmaceutical composition of the present invention varies according to body weight, age, gender, and health status of a patient, age of a patient, diet, administration time, administration method, excretion rate, and the severity of a disease. A daily dosage thereof may be administered parenterally in an amount of preferably 0.01 to 50 mg, and more preferably 0.1 mg to 30 mg per 1 kg of body weight a day based on HSA-Slit3 LRRD2, and a daily dosage thereof may be administered orally in a single dose or multiple doses in an amount of preferably 0.01 to 100 mg, and more preferably 0.01 to 10 mg per 1 kg of body weight a day based on the HSA-Slit3 LRRD2 of the present invention. However, since the effective amount may be increased or decreased depending on the administration route, the severity of obesity, gender, body weight, age, and the like, the dosage is not intended to limit the scope of the present invention in any way.

The pharmaceutical composition of the present invention may be used either alone or in combination with surgery, radiation therapy, hormone therapy, chemotherapy, and methods using a biological response modifier.

The pharmaceutical composition of the present invention may also be provided as a formulation for external application. When the pharmaceutical composition for preventing and treating a muscle disease according to the present invention is used as a preparation for external application to the skin, the pharmaceutical composition may additionally contain auxiliary agents typically used in the dermatology field, such as any other ingredients typically used in the preparation for external application to the skin, such as a fatty substance, an organic solvent, a solubilizing agent, a thickener and a gelling agent, a softener, an antioxidant, a suspending agent, a stabilizer, a foaming agent, an odorant, a surfactant, water, an ionic emulsifier, a non-ionic emulsifier, a filler, a metal ion blocking agent, a chelating agent, a preservative, a vitamin, a blocking agent, a wetting agent, an essential oil, a dye, a pigment, a hydrophilic active agent, a lipophilic active agent, or a lipid vesicle. In addition, the ingredients may be introduced in an amount generally used in the dermatology field.

When the pharmaceutical composition for preventing and treating a muscle disease according to the present invention is provided as a preparation for external application to the skin, the pharmaceutical composition may be in the form of a formulation such as an ointment, a patch, a gel, a cream, and an aerosol, but is not limited thereto.

It is preferred that the muscle disease of the present invention is a muscle disease caused by muscular function deterioration, muscle wasting, or muscle degeneration and is a disease reported in the art, and specifically, it is more preferred that the muscle disease of the present invention is one or more selected from the group consisting of atony, muscular atrophy, muscular dystrophy, muscle degeneration, myasthenia gravis, cachexia, and sarcopenia, but the muscle disease is not limited thereto.

The muscle wasting or degeneration occurs for reasons such as congenital factors, acquired factors, and aging, and the muscle wasting is characterized by a gradual loss of muscle mass, and weakening and degeneration of a muscle, particularly, a skeletal muscle or a voluntary muscle and a cardiac muscle.

More specifically, the muscle comprehensively refers to a sinew, a muscle, and a tendon, and the muscular function or muscle function means an ability to exert a force by contraction of the muscle, and includes: muscular strength in which the muscle can exert the maximum contraction force in order to withstand the resistance; muscular endurance strength which is an ability to exhibit how long or how many times the muscle can repeat the contraction and relaxation to a given weight; and quickness which is an ability to exert a strong force within a short period of time. The muscular function is proportional to the muscle mass, and the term "improvement of muscular function" refers to the improvement of the muscular function in a more positive direction.

The present invention also provides a health functional food composition comprising albumin-bound LRRD2 of the Slit3 protein for prevention or alleviation of a muscle disease. Since the composition of an active ingredient included in the health functional food composition of the present invention and effects thereof are the same as those for the above-described pharmaceutical composition, the description thereof will be omitted.

The health functional food composition according to the present invention can be prepared in various forms by typical methods known in the art. A general food can be prepared by adding the HSA-Slit3 LRRD2 fusion protein of the present invention to, without being limited to, a beverage (including an alcoholic beverage), fruit and a processed food thereof (for example: canned fruit, bottled food, jam, marmalade, and the like), fish, meat and processed food thereof (for example: ham, sausage, corned beef, and the like), bread and noodles (for example: thick wheat noodles, buckwheat noodles, instant noodles, spaghetti, macaroni, and the like), fruit juice, various drinks, cookies, wheat-gluten, dairy products (for example: butter, cheese, and the like), edible vegetable oils, margarine, vegetable protein, retort foods, frozen food and various seasonings (for example: soybean paste, soy sauce, sauce, and the like), and the like. In addition, a nutritional supplement can be prepared by adding the HSA-Slit3 LRRD2 fusion protein of the present invention to, without being limited to, a capsule, a tablet, a pill, and the like. Furthermore, for a health functional food, for example, the HSA-Slit3 LRRD2 fusion protein of the present invention itself is prepared in the form of, without being limited to, tea, juice, and drinks and can be taken by being processed into a liquid, granules, a capsule, and a powder so as to be able to be drunk (health beverage). Further, the HSA-Slit3 LRRD2 fusion protein of the present invention can be used and prepared in the form of a powder or a concentrated liquid so as to be used in the form of a food additive. In addition, the food functional composition of the present invention can be prepared in the form of a composition by mixing the HSA-Slit3 LRRD2 fusion protein of the present invention with an active ingredient known to have effects of preventing a muscle disease and improving muscular function.

When the HSA-Slit3 LRRD2 fusion protein of the present invention is used as a health beverage, the health beverage composition can contain various flavoring agents or natural carbohydrates, and the like as additional ingredients, such as a typical beverage. The above-described natural carbohydrates may be monosaccharides such as glucose and fructose; disaccharides such as maltose and sucrose; polysaccharides such as dextrin and cyclodextrin; and sugar alcohols such as xylitol, sorbitol, and erythritol. As a sweetener, it is possible to use a natural sweetener such as thaumatin and stevia extract; a synthetic sweetener such as saccharin and aspartame, and the like. The proportion of the natural carbohydrate is generally about 0.01 to 0.04 g, and preferably about 0.02 to 0.03 g per 100 mL of the composition of the present invention.

Furthermore, the HSA-Slit3 LRRD2 fusion protein of the present invention may be contained as an active ingredient of a food composition for preventing a muscle disease and improving muscular function, and the amount thereof is an amount effective to achieve an action for preventing a muscle disease and improving a muscular function and is not particularly limited, but is preferably 0.01 to 100 wt% based on the total weight of the entire composition. The health functional food composition of the present invention can be prepared by mixing the HSA-Slit3 LRRD2 fusion protein with other active ingredients known to have effects of preventing a muscle disease and improving a muscular function.

In addition to the aforementioned ingredients, the health functional food composition of the present invention may contain various nutrients, vitamins, electrolytes, flavoring agents, coloring agents, pectic acid, salts of pectic acid, alginic acid, salts of alginic acid, organic acids, protective colloidal thickeners, pH adjusting agents, stabilizers, preservatives, glycerin, alcohols, carbonate agents, and the like. In addition, the health food of the present invention may contain flesh for preparing natural fruit juice, fruit juice beverages, or vegetable beverages. These ingredients may be used either alone or in mixtures thereof. The proportion of these additives is not significantly important, but is generally selected within a range of 0.01 to 0.1 part by weight per 100 parts by weight of the composition of the present invention.

Further, the present invention provides a cosmetic composition comprising an HSA-Slit3 LRRD2 fusion protein for improving muscle function. Since the composition of an active ingredient included in the cosmetic composition of the present invention and effects thereof are the same as those for the above-described pharmaceutical composition, the description thereof will be omitted.

The cosmetic composition is not particularly limited, but may be used for external application to the skin.

The cosmetic composition of the present invention contains the HSA-Slit3 LRRD2 fusion protein as an active ingredient, and may be prepared in the form of a basic cosmetic composition (a lotion, a cream, an essence, a cleanser such as cleansing foam and cleansing water, a pack, and a body oil), a coloring cosmetic composition (a foundation, a lip-stick, a mascara, and a make-up base), a hair product composition (a shampoo, a rinse, a hair conditioner, and a hair gel), a soap, and the like with dermatologically acceptable excipients.

The excipient may include, for example, but not limited thereto, a skin softener, a skin infiltration enhancer, a coloring agent, an odorant, an emulsifier, a thickener, and a solvent. Further, it is possible to additionally include a fragrance, a pigment, a disinfectant, an antioxidant, a preservative, a moisturizer and the like, and to include a thickening agent, inorganic salts, a synthetic polymer material, and the like for improving physical properties. For example, when a cleanser and a soap are prepared by using the cosmetic composition of the present invention, the cleanser and the soap may be easily prepared by adding the HSA-Slit3 LRRD2 fusion protein to a typical cleanser and soap base. When cream is prepared, the cream may be prepared by adding the HSA-Slit3 LRRD2 fusion protein to a general oil-in-water (O/W) cream base. It is possible to further add a fragrance, a chelating agent, a pigment, an antioxidant, a preservative, and the like, and synthetic or natural materials such as proteins, minerals or vitamins for improving physical properties thereto.

The content of the HSA-Slit3 LRRD2 fusion protein contained in the cosmetic composition of the present invention is, but not limited to, preferably 0.001 to 10 wt%, and more preferably 0.01 to 5 wt%, based on the total weight of the entire composition. When the content is less than 0.001 wt%, desired effects cannot be expected, and when the content exceeds 10 wt%, it may be difficult to prepare the cosmetic composition of the present invention for reasons such as safety or formulation.

The present invention also provides a feed additive comprising albumin-bound LRRD2 of the Slit3 protein for improving muscle function. Since the composition of an active ingredient included in the feed additive of the present invention and effects thereof are the same as those for the above-described pharmaceutical composition, the description thereof will be omitted.

The feed additive of the present invention corresponds to a supplementary feed under the Control of Livestock and Fish Feed Act.

As used herein, the term "feed" may refer to any natural or artificial formula, one-meal, and the like, or a component of the one-meal for an animal to eat, ingest, and digest, or suitable for that.

The type of feed described above is not particularly limited, and a feed typically used in the art may be used. Non-limiting examples of the feed include vegetable feeds such as cereals, roots and fruits, food processing by-products, algae, fibers, pharmaceutical by-products, fats and oils, starches, gourds or grain by-products; and animal feeds such as proteins, inorganic substances, fats and oils, minerals, single cell proteins, animal plankton or foods. These feeds may be used alone or in mixtures of two or more thereof.

In addition, the feed additive may additionally contain a carrier that is acceptable to a unit animal. In the present invention, the feed additive may be used as it is, or a known carrier, stabilizer and the like may be added, various nutrients such as vitamins, amino acids, and minerals, antioxidants, other additives, and the like may be added, if necessary, and the shape thereof may be in a suitable state such as a powder, granules, a pellet, and a suspension. When the feed additive of the present invention is supplied, the feed additive may be supplied to a unit animal alone or in mixtures with the feed.

The present invention also provides a composition comprising albumin-bound LRRD2 of the Slit3 protein for improving the *in vivo* half-life of LRRD2 of the Slit3 protein.

Hereinafter, the present invention will be described in more detail through Examples. These Examples are only for exemplifying the present invention, and it should be obvious to a person with ordinary skill in the art that the scope of the present invention is not to be interpreted as being limited by these Examples.

The abbreviations used in the examples and meanings thereof are as shown in the following Table 1.

[Table 1]

| CL | Systemic plasma clearance |
|---|---|
| $T_{1/2}$ | Terminal half-life |
| $V_{ss}$ | Steady state volume of distribution |
| IV | Intravenous |
| PO | Per oral |
| $Cm_{ax}$ | Maximum plasma concentration observed |
| $T_{max}$ | Time to Cmax |
| $AUC_{0-\infty}$ | Total area under the plasma concentration time curve from zero to infinity |
| $AUC_{0-t}$ | Area under the plasma concentration time curve from zero to the last quantifiable time point |
| MRT | Mean residence time |
| BA | Estimated bioavailability |
| BQL | Below Quantification Level |

[Example 1]

**Preparation of HSA-Slit3 LRRD2 fusion protein**

Expression was performed by transforming Expi293F suspension cells with 1.6 mg/ml PC DNA3.1 vector SP cystatin S-HSA-Slit3 LRR D2-FLAG DNA. After cells were cultured to 4.5 to $5 \times 10^6$ cells/ml in 125 ml of a 293F cell suspension and only the medium was replaced with a new medium, transfection was performed by reacting 400 $\mu$l of Expifectamine with 7.5 ml of (A Sample) at room temperature for 5 minutes, reacting 150 ug of DNA with 7.5 ml of Opti-mem (B Sample) at room temperature for 5 minutes, and then mixing A and B Samples to react A and B Samples at room temperature for 20 minutes. After 24 hours, cells were treated by mixing Enhancers 1 and 2, and then cultured for 7 days.

After cells were precipitated from the culture solution cultured for 7 days using a centrifuge at 4°C and 800 rpm for 20 minutes, the supernatant was filtered with a 0.22 $\mu$m filter manufactured by Corning and used. As a resin, an anti-FLAG resin manufactured by Sigma was used. 1.2 ml of the resin was respectively used, and purification was performed at 1 ml/min at 4°C. A washing buffer using Tris glycine (TBS, pH 7.4) was flowed in an amount which is 20-fold higher than that of the resin. For elution, 200 $\mu$l of a FLAG peptide manufactured by Sigma-Aldrich and 9.8 ml of TBS were mixed and used, 8 pieces of 500 $\mu$l per fraction were obtained, protein fractions were collected, concentrated by changing the buffer to DPBS, and then the concentration was measured.

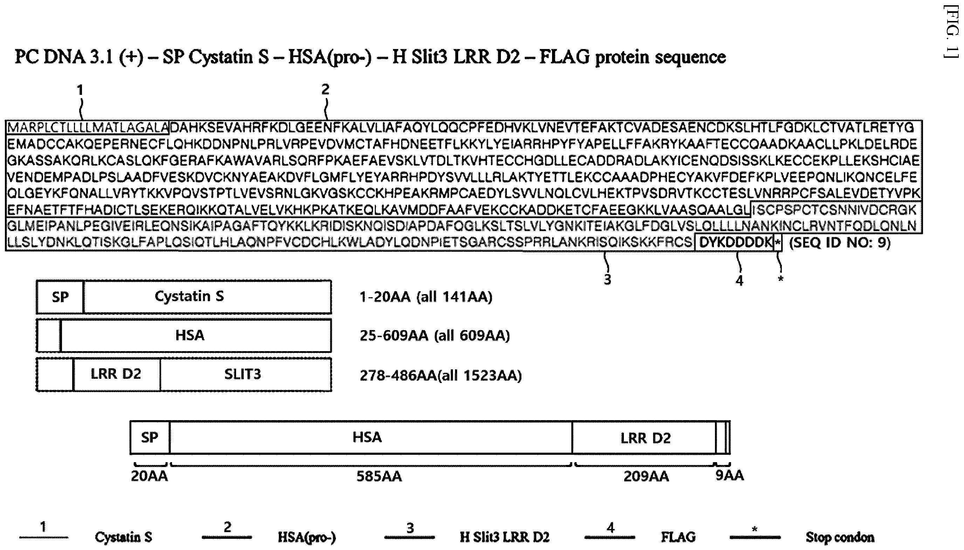

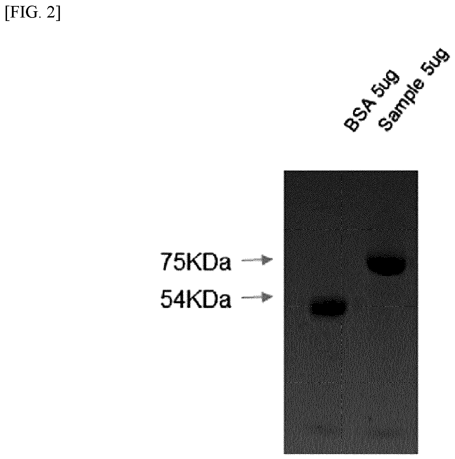

FIG. 2 illustrates the results of performing SDS-PAGE after isolating and purifying the fusion protein by the above process, confirming that the size of the fusion protein illustrated in FIG. 1, which was prepared in the present example, was 75 KDa.

[Example 2]

**Confirmation of receptor binding ability of various forms of HSA-Slit3 LRRD2 fusion proteins**

2-1. Preparation of various forms of HSA-Slit3 LRRD2 fusion proteins

**Based on the preparation method of Example 1, 12 types of various HSA-**Slit3 LRRD2 fusion proteins were prepared as shown in the following Table 2. As a linker, (GGGGS)$_3$ (SEQ ID NO: 6) was used.

[Table 2]

| Type of fusion protein | Terminus bound to HSA | Presence or absence of linker | Type of LRRD2 | Final form |
|---|---|---|---|---|
| LRRD2-1 | N terminus | None | Fragment (68 a.a.) | HSA-Fragment LRRD2 |
| LRRD2-2 | N terminus | None | Intermediate (130 a.a) | HSA-Intermediate LRRD2 |
| LRRD2-3 | N terminus | None | Full-length (209 a.a) | HSA-Full-length LRRD2 |
| LRRD2-4 | N terminus | Present | Fragment (68 a.a.) | HSA-Linker-Fragment LRRD2 |
| LRRD2-5 | N terminus | Present | Intermediate (130 a.a) | HSA-Linker-Intermediate LRRD2 |
| LRRD2-6 | N terminus | Present | Full-length (209 a.a) | HSA-Linker-Full-length LRRD2 |
| LRRD2-7 | C terminus | None | Fragment (68 a.a.) | Fragment LRRD2-HSA |
| LRRD2-8 | C terminus | None | Intermediate (130 a.a) | Intermediate LRRD2-HSA |
| LRRD2-9 | C terminus | None | Full-length (209 a.a) | Full-length LRRD2-HSA |
| LRRD2-10 | C terminus | Present | Fragment (68 a.a.) | Fragment LRRD2-Linker-HSA |
| LRRD2-11 | C terminus | Present | Intermediate (130 a.a) | Intermediate LRRD2-Linker-HSA |
| LRRD2-12 | C terminus | Present | Full-length (209 a.a) | Full-length LRRD2-Linker-HSA |

The amino acid sequences of the 12 types of HSA-Slit3LRRD2 fusion proteins are shown in Table 3.

[Table 3]

| Type of fusion protein | Amino acid sequence | SEQ ID NO |
|---|---|---|
| LRRD2-1 | MMARPLCTLLLLMATLAGALADAHKSEVA HRFKDLGEENFKALVLIAFAQYLQQCPFED HVKLVNEVTEFAKTCVADESAENCDKSLHT LFGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVAR LSQRFPKAEFAEVSKLVTDLTKVHTECCHG DLLECADDRADLAKYICENQDSISSKLKECC EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL GKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHKPKATKEQLKAVMDD FAAFVEKCCKADDKETCFAEEGKKLVAASQ AALGLLTSLVLYGNKITEIAKGLFDGLVSLQ LLLLNANKINCLRVNTFQDLQNLNLLSLYD NKLQTISKGLFADYKDDDDK | 7 |
| LRRD2-2 | MARPLCTLLLLMATLAGALADAHKSEVAH RFKDLGEENFKALVLIAFAQYLQQCPFEDH VKLVNEVTEFAKTCVADESAENCDKSLHTL FGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVAR LSQRFPKAEFAEVSKLVTDLTKVHTECCHG DLLECADDRADLAKYICENQDSISSKLKECC EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL | 8 |

(continued)

| Type of fusion protein | Amino acid sequence | SEQ ID NO |
|---|---|---|
| | GKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHKPKATKEQLKAVMDD FAAFVEKCCKADDKETCFAEEGKKLVAASQ AALGLIVEIRLEQNSIKAIPAGAFTQYKKLKR IDISKNQISDIAPDAFQGLKSLTSLVLYGNKI TEIAKGLFDGLVSLQLLLLNANKINCLRVNT FQDLQNLNLLSLYDNKLQTISKGLFAPLQSI QTLHLAQNPDYKDDDDK | |
| LRRD2-3 | MARPLCTLLLLMATLAGALADAHKSEVAH RFKDLGEENFKALVLIAFAQYLQQCPFEDH VKLVNEVTEFAKTCVADESAENCDKSLHTL FGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVAR LSQRFPKAEFAEVSKLVTDLTKVHTECCHG DLLECADDRADLAKYICENQDSISSKLKECC EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL GKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHKPKATKEQLKAVMDD FAAFVEKCCKADDKETCFAEEGKKLVAASQ AALGLISCPSPCTCSNNIVDCRGKGLMEIPA NLPEGIVEIRLEQNSIKAIPAGAFTQYKKLKR IDISKNQISDIAPDAFQGLKSLTSLVLYGNKI TEIAKGLFDGLVSLQLLLLNANKINCLRVNT FQDLQNLNLLSLYDNKLQTISKGLFAPLQSI QTLHLAQNPFVCDCHLKWLADYLQDNPIET SGARCSSPRRLANKRISQIKSKKFRCSDYKD DDDK | 9 |

(continued)

| Type of fusion protein | Amino acid sequence | SEQ ID NO |
|---|---|---|
| LRRD2-4 | MARPLCTLLLLMATLAGALADAHKSEVAH RFKDLGEENFKALVLIAFAQYLQQCPFEDH VKLVNEVTEFAKTCVADESAENCDKSLHTL FGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVAR LSQRFPKAEFAEVSKLVTDLTKVHTECCHG DLLECADDRADLAKYICENQDSISSKLKECC EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL GKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHPKATKEQLKAVMDD FAAFVEKCCKADDKETCFAEEGKKLVAASQ AALGL<u>GGGGSGGGGSGGGGS</u>LTSLVLYGN KITEIAKGLFDGLVSLQLLLLNANKINCLRV NTFQDLQNLNLLSLYDNKLQTISKGLFADY KDDDDK | 10 |

(continued)

| Type of fusion protein | Amino acid sequence | SEQ ID NO |
|---|---|---|
| LRRD2-5 | MARPLCTLLLLMATLAGALADAHKSEVAH RFKDLGEENFKALVLIAFAQYLQQCPFEDH VKLVNEVTEFAKTCVADESAENCDKSLHTL FGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVAR LSQRFPKAEFAEVSKLVTDLTKVHTECCHG DLLECADDRADLAKYICENQDSISSKLKECC EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL GKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHPKATKEQLKAVMDD FAAFVEKCCKADDKETCFAEEGKKLVAASQ AALGL<u>GGGGSGGGGSGGGGS</u>IVEIRLEQNSI KAIPAGAFTQYKKLRIDISKNQISDIAPDAF QGLKSLTSLVLYGNKITEIAKGLFDGLVSLQ LLLLNANKINCLRVNTFQDLQNLNLLSLYD NKLQTISKGLFAPLQSIQTLHLAQNPDYKDD DDK | 11 |
| LRRD2-6 | MARPLCTLLLLMATLAGALADAHKSEVAH RFKDLGEENFKALVLIAFAQYLQQCPFEDH VKLVNEVTEFAKTCVADESAENCDKSLHTL FGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVAR LSQRFPKAEFAEVSKLVTDLTKVHTECCHG DLLECADDRADLAKYICENQDSISSKLKECC | 12 |

(continued)

| Type of fusion protein | Amino acid sequence | SEQ ID NO |
|---|---|---|
|  | EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL GKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHKPKATKEQLKAVMDD FAAFVEKCCKADDKETCFAEEGKKLVAASQ AALGLGGGGSGGGGSGGGGSISCPSPCTCSN NIVDCRGKGLMEIPANLPEGIVEIRLEQNSIK AIPAGAFTQYKKLKRIDISKNQISDIAPDAFQ GLKSLTSLVLYGNKITEIAKGLFDGLVSLQL LLLNANKINCLRVNTFQDLQNLNLLSLYDN KLQTISKGLFAPLQSIQTLHLAQNPFVCDCH LKWLADYLQDNPIETSGARCSSPRRLANKRI SQIKSKKFRCSDYKDDDDK |  |
| LRRD2-7 | MARPLCTLLLLMATLAGALALTSLVLYGNK ITEIAKGLFDGLVSLQLLLLNANKINCLRVN TFQDLQNLNLLSLYDNKLQTISKGLFADAH KSEVAHRFKDLGEENFKALVLIAFAQYLQQ CPFEDHVKLVNEVTEFAKTCVADESAENCD KSLHTLFGDKLCTVATLRETYGEMADCCAK QEPERNECFLQHKDDNPNLPRLVRPEVDVM CTAFHDNEETFLKKYLYEIARRHPYFYAPEL LFFAKRYKAAFTECCQAADKAACLLPKLDE LRDEGKASSAKQRLKCASLQKFGERAFKA WAVARLSQRFPKAEFAEVSKLVTDLTKVHT ECCHGDLLECADDRADLAKYICENQDSISSK LKECCEKPLLEKSHCIAEVENDEMPADLPSL AADFVESKDVCKNYAEAKDVFLGMFLYEY ARRHPDYSVVLLLRLAKTYETTLEKCCAAA DPHECYAKVFDEFKPLVEEPQNLIKQNCELF EQLGEYKFQNALLVRYTKKVPQVSTPTLVE VSRNLGKVGSKCCKHPEAKRMPCAEDYLS VVLNQLCVLHEKTPVSDRVTKCCTESLVNR RPCFSALEVDETYVPKEFNAETFTFHADICT LSEKERQIKKQTALVELVKHKPKATKEQLK AVMDDFAAFVEKCCKADDKETCFAEEGKK LVAASQAALGL**GGGGSGGGGSGGGGS**DY KDDDDK | 13 |

(continued)

| Type of fusion protein | Amino acid sequence | SEQ ID NO |
|---|---|---|
| LRRD2-8 | MARPLCTLLLLMATLAGALAIVEIRLEQNSI KAIPAGAFTQYKKLKRIDISKNQISDIAPDAF QGLKSLTSLVLYGNKITEIAKGLFDGLVSLQ LLLLNANKINCLRVNTFQDLQNLNLLSLYD NKLQTISKGLFAPLQSIQTLHLAQNPDAHKS EVAHRFKDLGEENFKALVLIAFAQYLQQCP FEDHVKLVNEVTEFAKTCVADESAENCDKS LHTLFGDKLCTVATLRETYGEMADCCAKQ EPERNECFLQHKDDNPNLPRLVRPEVDVMC TAFHDNEETFLKKYLYEIARRHPYFYAPELL FFAKRYKAAFTECCQAADKAACLLPKLDEL RDEGKASSAKQRLKCASLQKFGERAFKAW AVARLSQRFPKAEFAEVSKLVTDLTKVHTE CCHGDLLECADDRADLAKYICENQDSISSKL KECCEKPLLEKSHCIAEVENDEMPADLPSLA ADFVESKDVCKNYAEAKDVFLGMFLYEYA RRHPDYSVVLLLRLAKTYETTLEKCCAAAD PHECYAKVFDEFKPLVEEPQNLIKQNCELFE QLGEYKFQNALLVRYTKKVPQVSTPTLVEV SRNLGKVGSKCCKHPEAKRMPCAEDYLSV VLNQLCVLHEKTPVSDRVTKCCTESLVNRR PCFSALEVDETYVPKEFNAETFTFHADICTLS EKERQIKKQTALVELVKHKPKATKEQLKAV MDDFAAFVEKCCKADDKETCFAEEGKKLV AASQAALGL**GGGGSGGGGSGGGG**SDYKD DDDK | 14 |

(continued)

| Type of fusion protein | Amino acid sequence | SEQ ID NO |
|---|---|---|
| LRRD2-9 | MARPLCTLLLLMATLAGALAISCPSPCTCSN NIVDCRGKGLMEIPANLPEGIVEIRLEQNSIK AIPAGAFTQYKKLKRIDISKNQISDIAPDAFQ GLKSLTSLVLYGNKITEIAKGLFDGLVSLQL LLLNANKINCLRVNTFQDLQNLNLLSLYDN KLQTISKGLFAPLQSIQTLHLAQNPFVCDCH LKWLADYLQDNPIETSGARCSSPRRLANKRI SQIKSKKFRCSDAHKSEVAHRFKDLGEENF KALVLIAFAQYLQQCPFEDHVKLVNEVTEF AKTCVADESAENCDKSLHTLFGDKLCTVAT LRETYGEMADCCAKQEPERNECFLQHKDD NPNLPRLVRPEVDVMCTAFHDNEETFLKKY LYEIARRHPYFYAPELLFFAKRYKAAFTECC QAADKAACLLPKLDELRDEGKASSAKQRL KCASLQKFGERAFKAWAVARLSQRFPKAEF AEVSKLVTDLTKVHTECCHGDLLECADDRA DLAKYICENQDSISSKLKECCEKPLLEKSHCI AEVENDEMPADLPSLAADFVESKDVCKNY AEAKDVFLGMFLYEYARRHPDYSVVLLLRL AKTYETTLEKCCAAADPHECYAKVFDEFKP LVEEPQNLIKQNCELFEQLGEYKFQNALLVR YTKKVPQVSTPTLVEVSRNLGKVGSKCCKH PEAKRMPCAEDYLSVVLNQLCVLHEKTPVS DRVTKCCTESLVNRRPCFSALEVDETYVPK EFNAETFTFHADICTLSEKERQIKKQTALVE LVKHKPKATKEQLKAVMDDFAAFVEKCCK ADDKETCFAEEGKKLVAASQAALGL**GGGG SGGGGSGGGGS**DYKDDDDK | 15 |
| LRRD2-10 | MARPLCTLLLLMATLAGALALTSLVLYGNK ITEIAKGLFDGLVSLQLLLLLNANKINCLRVN | 16 |

(continued)

| Type of fusion protein | Amino acid sequence | SEQ ID NO |
|---|---|---|
| | TFQDLQNLNLLSLYDNKLQTISKGLFA*GGG* *GSGGGGSGGGGS*DAHKSEVAHRFKDLGEE NFKALVLIAFAQYLQQCPFEDHVKLVNEVT EFAKTCVADESAENCDKSLHTLFGDKLCTV ATLRETYGEMADCCAKQEPERNECFLQHK DDNPNLPRLVRPEVDVMCTAFHDNEETFLK KYLYEIARRHPYFYAPELLFFAKRYKAAFTE CCQAADKAACLLPKLDELRDEGKASSAKQ RLKCASLQKFGERAFKAWAVARLSQRFPKA EFAEVSKLVTDLTKVHTECCHGDLLECADD RADLAKYICENQDSISSKLKECCEKPLLEKS HCIAEVENDEMPADLPSLAADFVESKDVCK NYAEAKDVFLGMFLYEYARRHPDYSVVLL LRLAKTYETTLEKCCAAADPHECYAKVFDE FKPLVEEPQNLIKQNCELFEQLGEYKFQNAL LVRYTKKVPQVSTPTLVEVSRNLGKVGSKC CKHPEAKRMPCAEDYLSVVLNQLCVLHEK TPVSDRVTKCCTESLVNRRPCFSALEVDETY VPKEFNAETFTFHADICTLSEKERQIKKQTA LVELVKHKPKATKEQLKAVMDDFAAFVEK CCKADDKETCFAEEGKKLVAASQAALGL**G** **GGGSGGGGSGGGGS**DYKDDDDK | |

(continued)

| Type of fusion protein | Amino acid sequence | SEQ ID NO |
|---|---|---|
| LRRD2-11 | MARPLCTLLLLMATLAGALAIVEIRLEQNSIKAIPAGAFTQYKKLKRIDISKNQISDIAPDAFQGLKSLTSLVLYGNKITEIAKGLFDGLVSLQLLLLNANKINCLRVNTFQDLQNLNLLSLYDNKLQTISKGLFAPLQSIQTLHLAQNP<u>GGGGSGGGGSGGGGS</u>DAHKSEVAHRFKDLGEENFKALVLIAFAQYLQQCPFEDHVKLVNEVTEFAKTCVADESAENCDKSLHTLFGDKLCTVATLRETYGEMADCCAKQEPERNECFLQHKDDNPNLPRLVRPEVDVMCTAFHDNEETFLKKYLYEIARRHPYFYAPELLFFAKRYKAAFTECCQAADKAACLLPKLDELRDEGKASSAKQRLKCASLQKFGERAFKAWAVARLSQRFPKAEFAEVSKLVTDLTKVHTECCHGDLLECADDRADLAKYICENQDSISSKLKECCEKPLLEKSHCIAEVENDEMPADLPSLAADFVESKDVCKNYAEAKDVFLGMFLYEYARRHPDYSVVLLLRLAKTYETTLEKCCAAADPHECYAKVFDEFKPLVEEPQNLIKQNCELFEQLGEYKFQNALLVRYTKKVPQVSTPTLVEVSRNLGKVGSKCCKHPEAKRMPCAEDYLSVVLNQLCVLHEKTPVSDRVTKCCTESLVNRRPCFSALEVDETYVPKEFNAETFTFHADICTLSEKERQIKKQTALVELVKHKPKATKEQLKAVMDDFAAFVEKCCKADDKETCFAEEGKKLVAASQAALGL**GGGGSGGGGSGGGGS**DYKDDDDK | 17 |
| LRRD2-12 | MARPLCTLLLLMATLAGALAISCPSPCTCSN | 18 |

(continued)

| Type of fusion protein | Amino acid sequence | SEQ ID NO |
|---|---|---|
| | NIVDCRGKGLMEIPANLPEGIVEIRLEQNSIK AIPAGAFTQYKKLKRIDISKNQISDIAPDAFQ GLKSLTSLVLYGNKITEIAKGLFDGLVSLQL LLLNANKINCLRVNTFQDLQNLNLLSLYDN KLQTISKGLFAPLQSIQTLHLAQNPFVCDCH LKWLADYLQDNPIETSGARCSSPRRLANKRI SQIKSKKFRCS<u>GGGGSGGGGSGGGGS</u>DAHK SEVAHRFKDLGEENFKALVLIAFAQYLQQC PFEDHVKLVNEVTEFAKTCVADESAENCDK SLHTLFGDKLCTVATLRETYGEMADCCAKQ EPERNECFLQHKDDNPNLPRLVRPEVDVMC TAFHDNEETFLKKYLYEIARRHPYFYAPELL FFAKRYKAAFTECCQAADKAACLLPKLDEL RDEGKASSAKQRLKCASLQKFGERAFKAW AVARLSQRFPKAEFAEVSKLVTDLTKVHTE CCHGDLLECADDRADLAKYICENQDSISSKL KECCEKPLLEKSHCIAEVENDEMPADLPSLA ADFVESKDVCKNYAEAKDVFLGMFLYEYA RRHPDYSVVLLLRLAKTYETTLEKCCAAAD PHECYAKVFDEFKPLVEEPQNLIKQNCELFE QLGEYKFQNALLVRYTKKVPQVSTPTLVEV SRNLGKVGSKCCKHPEAKRMPCAEDYLSV VLNQLCVLHEKTPVSDRVTKCCTESLVNRR PCFSALEVDETYVPKEFNAETFTFHADICTLS EKERQIKKQTALVELVKHKPKATKEQLKAV MDDFAAFVEKCCKADDKETCFAEEGKKLV AASQAALGL**GGGGSGGGGSGGGGS**DYKD DDDK | |
| *The underlined sequence in Table 3 is a GS linker linking HSA and LRRD2, and the bold sequence is a GS linker linking a sequence added to the C-terminus in order to express the fusion protein in its final form. | | |

2-2. Confirmation of receptor binding ability of various forms of HSA-Slit3 LRRD2 fusion proteins

Slit3 LRRD2 binds to the Robo1 or Robo2 receptor, and as a result, the β-catenin binding to the M-cadherin of myoblasts is released via the Slit-Robo system to activate the β-catenin and increase the expression of myogenin, and subsequently, the formation of muscles is promoted by inducing the differentiation of myoblasts. Therefore, in the present example, the receptor binding ability of the 12 types of HSA-Slit3 LRRD2 fusion proteins prepared in Example 2-1 was confirmed. The binding ability of 12 types of HSA-Slit3 LRRD2 fusion proteins to the Robo1 receptor was quantified using an ELISA system. Detailed conditions are as follows.

96-well microtiter plates (manufactured by NUNC) were coated with 12 types of HSA-Slit3 LRRD2 fusion proteins at 4°C for 18 hours at 0, 1, 10, 100, and 1000 nM per well, in consideration of the molecular weight. The coated material was washed three times using PBS containing 0.05% Tween 20 (PBST). Blocking was performed with PBST supplemented with 1% BSA at room temperature for 2 hours to block non-specific binding. The coated material was washed three times with PBST to remove a blocking buffer. After washing, 30 ug of a protein obtained from the corresponding osteoblastic cell line was allowed to adhere (lysis buffer: 0.5% NP40, 50 mM Tris pH 7.5, 150 mM NaCl, ImM EDTA, 0.2 mM NaF, 1 mM Na3VO4, 1 mM DTT, 1 mM PMSF, and a proteinase inhibitor cocktail) at room temperature for 2 hours. After washing three times with PBST, a Robo1 antibody (abcam: ab7279) diluted with 0.1% BSA at 1:1000 was adhered thereto at room temperature for 2 hours. After washing three times with PBST, an HRP-binding antibody (cell signaling: 7074) diluted with 0.1% BSA at 1:2000 was adhered thereto at room temperature for 2 hours. After washing five times with PBST, a reaction was performed with a TMB solution at 37°C for 30 minutes. To stop the reaction, 100 $\mu$l of 1 N $H_2SO_4$ was used, and absorbance was measured at 450 nm.

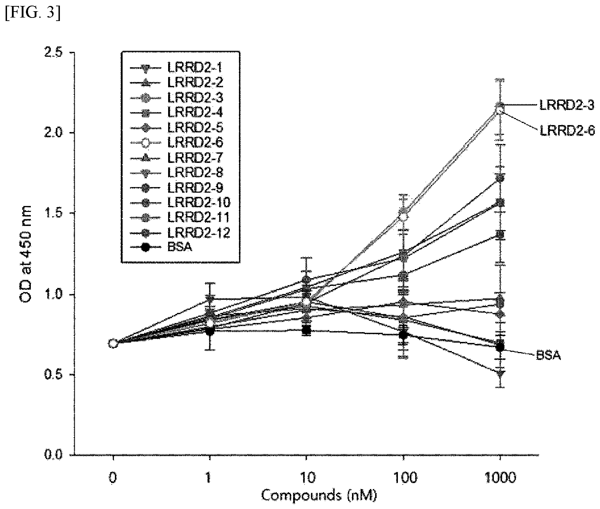

As a result, as illustrated in FIG. 3, it was confirmed that the receptor binding ability of LRRD2-3 and LRRD2-6 was the best.

[Example 3]

**Pharmacokinetic studies of Slit3 LRRD2 and HSA-Slit3 LRRD2 fusion proteins in mice**

In the present example, based on the results of Example 2, a pharmacokinetic study was conducted by selecting LRRD2-3, which has the best receptor binding ability.

A pharmacokinetic study is a part of new drug development processes, and aims to obtain information on the absorption, distribution, metabolism and excretion of a test drug by assessing changes in drug concentration in the body over time. In the present example, pharmacokinetic properties were confirmed in mice after a single intravenous administration of Slit3 LRRD2-3 and HSA-Slit3 LRRD2 fusion protein (LRRD2-3).

3-1. Chemicals and solvents

The carbamazepine used in this example was purchased from Sigma Aldrich, and HPLC grade acetonitrile and methanol were purchased from J.T. Baker.

3-2. Animals and administration conditions

In the present example, ICR-based male mice (6 weeks old, Orient Bio Co., Ltd., Seongnam, Republic of Korea) with a body weight ranging from 30 to 32.5 g were used. Mice were fasted for 4 hours before the experiment and fasting was maintained for up to 4 hours after administration. The breeding place was given 12 hours each of light and dark, and an appropriate temperature (20 to 25°C) and humidity (40 to 60%) were maintained.

[Table 4]

| Pharmacokinetic test | | |
|---|---|---|
| Administered material | Number of animals | Administration dose |
| Slit3 LRRD2 | 4 | 10 mg/kg |
| HSA-Slit3 LRRD2 (LRRD2-3) | 3 | 35 mg/kg |
| Total | 7 | - |

Slit3 LRRD2 was prepared by being dissolved in PBS at a dose of 1 mg/mL. HSA-Slit3 LRRD2 (LRRD2-3) was prepared by being dissolved in PBS at a dose of 3.5 mg/mL (1 mg/mL for Slit3 LRRD2) in consideration of the molecular weight. The dose was 10 mL/kg in both groups, and the prepared solution was administered through the left caudal vein.

3-3. Pharmacokinetic test

In the case of the pharmacokinetic test, fasted mice were administered Slit3 LRRD2 and HSA-Slit3 LRRD2 (LRRD2-3) at a dose of 10 mg/kg and 35 mg/kg, respectively, through the caudal vein. After administration, mice were fixed by hand at 0.05, 0.12, 0.33, 1, 3, 7, 10, 24, 48, and 72 hours, respectively, and then 70 $\mu$L of blood was collected from the right orbital venous plexus using heparin-coated capillary tubes. The collected blood was centrifuged for 5 minutes and then stored frozen at -20°C until plasma was isolated and analyzed.

3-4. Analysis Method

The concentration of Slit3 LRRD2 in plasma samples was quantified using an HPLC/MS/MS system. Before sample pretreatment, plasma samples were purified using Ni-NTA magnetic beads. After purified Slit3 LRRD2 and HSA-Slit3 LRRD2 (LRRD2-3) were denatured by adding 6M urea and 18 mM dithiothreitol (DTT) thereto, alkylation was induced using 225 mM iodine acetamide. Then, to obtain a signature peptide, 850 ng of recombinant porcine trypsin (V5117, Promega, Madison, WI, USA) was added thereto, and the resulting mixture was reacted in a water bath set at 37°C for 24 hours. After 50 $\mu$L of 3% formic acid dissolved in MeOH was added to 70 $\mu$L of a trypsin digestion product produced after the reaction, the mixed sample was suspended using a vortex mixer for 10 minutes, centrifuged at 13,500 rpm for 10 minutes, and 160 μL of the supernatant was taken and transferred to an analysis vessel, and 5 μL of the transferred supernatant was injected into an HPLC MSMS system to perform analysis.

Detailed analysis conditions are as follows.

- HPLC system: Agilent 1100 (Agilent Technologies, Santa Clara, CA)
- Column: ZORBAX® C$_8$ 3.5 μm, 2.1*50 mm (Agilent)
- Mobile phase:

A: 0.1% formic acid dissolved in distilled water
B: Acetonitrile
(Isocratic elution)

| Time | $0 \to 0.1 \to 1.0 \to 1.5 \to 2.5 \to 3 \to 5$ |
|---|---|
| B (%) | $5 \to 5 \to 5 \to 95 \to S5 \to 5 \to 5$ |

- Flow rate: 300 μL/min - Temperature: 20°C in column, and 10°C in autosampler tray
- Runtime: 5 minutes
- Detection: Tandem quadrupole mass spectrometer (API 4000, QTRAP®, Applied Biosystems/MDS SCIEX, Foster City, CA, USA)
- Curtain gas: 20 psi
- Ion source gas 1: 50 psi
- Ion source gas 2: 60 psi
- Ionspray voltage: 5500 V
- Temperature: 600°C
- Multiple-reaction-monitoring (MRM) mode: Positive

The molecular ions of a Silt3 LRRD2 signature peptide (P6) were fragmented by a collision energy of 23 V, and a collision gas was set to 'medium (8 psi)' in the equipment. Ions were detected in the ESI-positive MRM mode, and P6 was quantified from 587.97 to 491.50 in units of m/z. Detected peaks were integrated using Analyst software version 1.4.2 (Applied Biosystems/MDS SCIEX). A quantifiable range of Silt3 LRRD2 in plasma was 1 to 100 μg/mL, and that of HSA-Silt3 LRRD2 (LRRD2-3) was 3 to 100 μg/mL. In the corresponding analysis, Silt3 LRRD2 showed a peak retention time of 3.29 minutes.

3-5. Data analysis

The concentration of CNC00000 in plasma over time was determined using the LC-MS/MS analysis method described in Example 3-4, and pharmacokinetic parameters (PK parameters) were calculated using a non-compartmental analysis of WinNonlin® 4.2 (Pharsight Corp., Cary, NC, USA) software. The maximum concentration (C$_{max}$) and the maximum concentration arrival time (T$_{max}$) were temporally calculated from a curve according to the blood drug concentration vs. time, and the elimination rate constant (K$_e$) was calculated by a linear regression analysis in the terminal phase of the log scale. The half-life (T$_{1/2}$) was calculated by dividing LN2 by Ke, and an area under the curve of blood drug concentration vs. time (AUC$_{0-\infty}$) and an area under the curve of blood drug moment vs. time (AUMC$_{0-\infty}$) were calculated by the linear trapezoidal rule and the standard area extrapolation method. Clearance (CL) and steady state volume of distribution (Vss) were calculated by the following [Equation 1] to [Equation 3]:

[Equation 1]

$$CL = \frac{Dose}{AUC_{0-\infty}}$$

[Equation 2]

$$V_{ss} = MRT \times CL$$

[Equation 3]

$$MRT = \frac{AUMC_{0-\infty}}{AUC_{0-\infty}}$$

3-6. Results

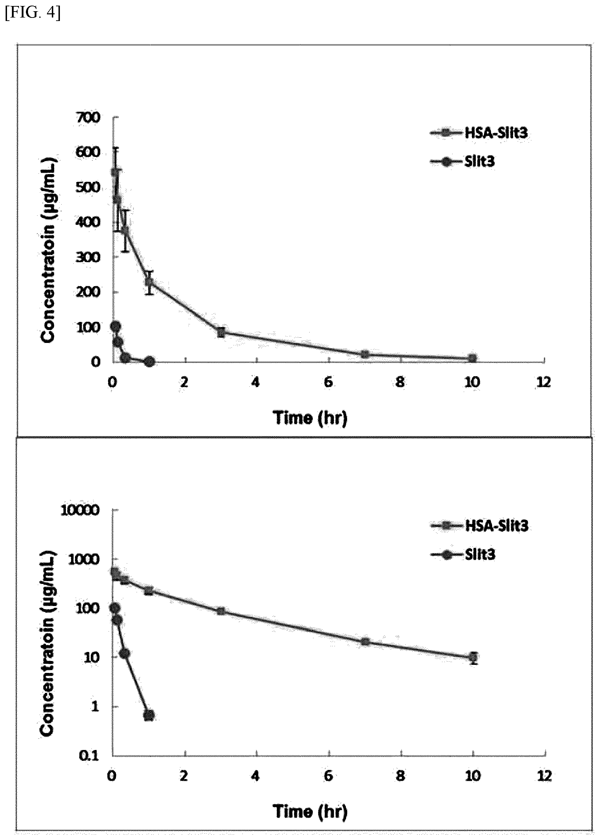

The concentrations of Slit3 LRRD2 and HSA-Slit3 LRRD2 (LRRD2-3) in plasma over time are shown in FIG. 4 and Tables 5 and 6, and pharmacokinetic parameters are shown in Table 6. The related parameters and all values were calculated for each individual and then averaged. Referring to the blood concentration pattern and animal experiment record over time, any abnormal populations were excluded from the data analysis, and the experimental group used for data analysis was set to have at least n = 3 or more.

[Table 5]

| Plasma concentration after intravenous administration of Slit3 | | | | | |
|---|---|---|---|---|---|
| Plasma concentration of Slit3 ($\mu$g/mL) | | | | | |
| Time (h) | #1 | #2 | #3 | #4 | mean | S.D. |
| 0.05 | 122 | 90.9 | 97.6 | 96.7 | 102 | 13.8 |
| 0.12 | 62.6 | 61.4 | 47.9 | 59.7 | 57.9 | 6.77 |
| 0.33 | 14.1 | 11.4 | 12.1 | 11.0 | 12.2 | 1.38 |
| 1 | 0.66 | 0.48 | 0.84 | 0.71 | 0.67 | 0.15 |
| 3 | BQL | BQL | BQL | BQL | 0.000 | - |
| 7 | BQL | BQL | BQL | BQL | 0.000 | - |
| 10 | BQL | BQL | BQL | BQL | 0.000 | - |
| 24 | BQL | BQL | BQL | BQL | 0.000 | - |
| *BQL: When it is less than the quantification limit, it is treated as "0". | | | | | |

[Table 6]

| Plasma concentration after intravenous administration of HSA-Slit3 (LRRD2-3) | | | | |
|---|---|---|---|---|
| Plasma concentration of Slit3 ($\mu$g/mL) | | | | |
| Time (h) | #6 | #7 | #8 | mean | S.D. |
| 0.05 | 587 | 460 | 577 | 541 | 70.6 |
| 0.12 | 539 | 366 | 480 | 462 | 87.9 |
| 0.33 | 355 | 327 | 441 | 374 | 59.4 |
| 1 | 217 | 199 | 262 | 226 | 32.4 |
| 3 | 82.8 | 73.6 | 98.9 | 85.1 | 12.8 |

(continued)

| Plasma concentration after intravenous administration of HSA-Slit3 (LRRD2-3) | | | | | |
|---|---|---|---|---|---|
| Plasma concentration of Slit3 ($\mu$g/mL) | | | | | |
| 7 | 18.9 | 18.5 | 23.4 | 20.3 | 2.72 |
| 10 | 9.71 | 7.64 | 12.5 | 9.95 | 2.44 |
| 24 | BQL | BQL | BQL | 0.000 | - |
| *BQL: When it is less than the quantification limit, it is treated as "0". | | | | | |

As confirmed in the following Table 7, the HSA-bound Slit3 LRRD2 (LRRD2-3) showed an approximately 14-fold improved half-life compared to Slit3 LRRD2.

[Table 7]

| Parameter | Slit3 LRRD2 | | HSA-Slit3 LRRD2 (LRRD2-3) | |
|---|---|---|---|---|
| | Average | S.D. | Average | S.D. |
| $T_{max}$(h) | 0.050 $\pm$ 0.000 | | 0.050 $\pm$ 0.000 | |
| $C_0$ ($\mu$g/mL) | 153.9 33.25 | | 607.8 59.87 | |
| $C_{max}$ ($\mu$g/mL) | 101.8 $\pm$ 13.79 | | 541.3 $\pm$ 70.61 | |
| $T_{1/2}$ (h) | 0.139 $\pm$ 0.012 | | 1.993 $\pm$ 0.147 | |
| $AUC_{all}$ ($\mu$g·h/mL) | 23.63 $\pm$ 2.534 | | 919.9 $\pm$ 131.2 | |
| $AUC_{inf}$ ($\mu$g·h/mL) | 23.77 $\pm$ 2.530 | | 948.1 $\pm$ 136.1 | |
| CL (mL/h/kg) | 424.0 $\pm$ 40.88 | | 10.69 $\pm$ 1.504 | |
| $V_{ss}$ (mL/kg) | 61.46 $\pm$ 7.368 | | 24.1 $\pm$ 3.293 | |

[Example 4]

**Confirmation of *in vivo* efficacy of HSA-bound Slit3 LRRD2**

9-week-old Balbc-nude mice subjected to an ovariectomy were treated with Slit3 LRRD2 to which albumin was not bound or HSA-Slit3 LRD2 fusion protein (LRRD2-3) for 4 weeks from the time when the mice became 11 weeks old. Each drug was administered by intravenous injection once daily, five times per week, and Slit3 LRRD2 and HSA-bound Slit3 LRRD2 (LRRD2-3) were injected daily in a dose of 10 mg and 37.13 mg, respectively (Slit3 LRRD2 corresponds to 10 mg daily). After the administration was completed, the soleus muscle was collected and the weight of the muscle was measured, and the results are shown in the following Table 8.

[Table 8]

| Group | Muscle mass (mg) of soleus muscle |
|---|---|
| Control (n = 8) | 5.89 $\pm$ 0.25 |
| Slit3 LRRD2 (n = 8) | 6.73 $\pm$ 0.20* |
| HSA-Slit3 LRRD2 fusion protein (LRRD2-3) (n = 8) | 6.80 $\pm$ 1.75* |
| *P < 0.05, vs. non-treated control | |

As shown in Table 8, both albumin-unbound Slit3 LRRD2 and HSA-bound Slit3 LRRD2 significantly increased the muscle mass of soleus muscle. However, HSA-bound Slit3 LRRD2 showed even stronger therapeutic efficacy than albumin-unbound Slit3 LRRD2.

[Industrial Applicability]

Since the albumin-bound LRRD2 of the Slit3 protein exhibits the same cytological efficacy as albumin-unbound LRRD2 of the Slit3 protein and has a significantly increased *in vivo* half-life compared to albumin-unbound LRRD2 of the Slit3 protein, bone-related diseases can be more effectively prevented or treated.

The national research and development projects supporting the present invention are as follows.

(1) [National research and development projects supporting the present invention]

[Project Identification Number] 2017-1229 (HI15C0377010017)
[Ministry Name] Ministry of Health and Welfare
[Research Management Agency] Korea Health Industry Development Institute
[Research Project Name] Disease Oriented Translational Research
[Research Title] Discovery of macronuclear cell secretion factors with bone formation promotion
[Contribution Rate] 75/100
[Administrative Organization] Asan Medical Center, Seoul
[Research Period] September 7, 2017 to September 6, 2018

(2) [National research and development projects supporting the present invention]

[Project Identification Number] 2013-2234 (HI13C1634060018)
[Ministry Name] Ministry of Health and Welfare
[Research Management Agency] Korea Health Industry Development Institute
[Research Project Name] Disease Oriented Translational Research
[Research Title] Pharmacokinetic Study of Slit3 LRRD2 and in vivo Toxicity Verification Using Slit3 TG Mice
[Contribution Rate] 25/100
[Administrative Organization] Industry & Academic Cooperation in Chungnam National University (IAC)
[Research Period] November 1, 2013 to June 30, 2019

## Claims

1. A fusion protein comprising albumin-bound LRRD2 of the Slit3 protein.

2. The fusion protein of claim 1, wherein the albumin is human serum albumin.

3. The fusion protein of claim 2, wherein the human serum albumin is bound to the N-terminus of the LRRD2 of the Slit3 protein.

4. The fusion protein of claim 3, wherein the human serum albumin comprises an amino acid sequence of SEQ ID NO: 2, and the LRRD2 of the Slit3 protein comprises an amino acid sequence of SEQ ID NO: 3.

5. The fusion protein of claim 1, further comprising a linker between the albumin and the LRRD2 of the Slit3 protein.

6. The fusion protein of claim 5, wherein the linker is (GGGGS)n, wherein n is an integer from 1 to 10.

7. A nucleic acid molecule encoding the fusion protein of any one of claims 1 to 6.

8. A recombinant vector comprising the nucleic acid molecule of claim 7.

9. A transformant comprising the recombinant vector of claim 6.

10. A method for preparing a fusion protein comprising albumin-bound LRRD2 of the Slit3 protein, the method comprising culturing the transformant of claim 9.

11. A pharmaceutical composition comprising the fusion protein of any one of claims 1 to 6 for prevention or treatment of a muscle disease.

12. The pharmaceutical composition of claim 11, wherein the pharmaceutical composition is administered as an injection.

13. The composition of claim 11, wherein the muscle disease is one or more selected from the group consisting of atony, muscular atrophy, muscular dystrophy, muscle degeneration, myasthenia gravis, cachexia, and sarcopenia.

14. A composition comprising the fusion protein of any one of claims 1 to 6 for improving the *in vivo* half-life of LRRD2 of the Slit3 protein.

[FIG. 1]

EP 3 950 707 A1

## PC DNA 3.1 (+) – SP Cystatin S – HSA(pro-) – H Slit3 LRR D2 – FLAG protein sequence

1        2

MARPLCTLLLLMATLAGALADAHKSEVAHRFKDLGEENFKALVLIAFAQYLQQCPFEDHVKLVNEVTEFAKTCVADESAENCDKSLHTLFGDKLCTVATLRETYG
EMADCCAKQEPERNECFLQHKDDNPNLPRLVRPEVDVMCTAFHDNEETFLKKYLYEIARRHPYFYAPELLFFAKRYKAAFTECCQAADKAACLLPKLDELRDE
GKASSAKQRLKCASLQKFGERAFKAWAVARLSQRFPKAEFAEVSKLVTDLTKVHTECCHGDLLECADDRADLAKYICENQDSISSKLKECCEKPLLEKSHCIAE
VENDEMPADLPSLAADFVESKDVCKNYAEAKDVFLGMFLYEYARRHPDYSVVLLLRLAKTYETTLEKCCAAADPHECYAKVFDEFKPLVEEPQNLIKQNCELFE
QLGEYKFQNALLVRYTKKVPQVSTPTLVEVSRNLGKVGSKCCKHPEAKRMPCAEDYLSVVLNQLCVLHEKTPVSDRVTKCCTESLVNRRPCFSALEVDETYVPK
EFNAETFTFHADICTLSEKERQIKKQTALVELVKHKPKATKEQLKAVMDDFAAFVEKCCKADDKETCFAEEGKKLVAASQAALGLISCPSPCTCSNNIVDCRGK
GLMEIPANLPEGIVEIRLEQNSIKAIPAGAFTQYKKLKRIDISKNQISDIAPDAFQGLKSLTSLVLYGNKITEIAKGLFDGLVSLQLLLLNANKINCLRVNTFQDLQNLN
LLSLYDNKLQTISKGLFAPLQSIQTLHLAQNPFVCDCHLKWLADYLQDNPIETSGARCSSPRRLANKRISQIKSKKFRCS DYKDDDDK* (SEQ ID NO: 9)

3        4   *

| | | | |
|---|---|---|---|
| SP | Cystatin S | | 1-20AA (all 141AA) |
| | HSA | | 25-609AA (all 609AA) |
| | LRR D2 | SLIT3 | 278-486AA(all 1523AA) |

| SP | HSA | LRR D2 | |
|---|---|---|---|
| 20AA | 585AA | 209AA | 9AA |

1 —— Cystatin S   2 —— HSA(pro-)   3 —— H Slit3 LRR D2   4 —— FLAG   * —— Stop condon

74

[FIG. 2]

[FIG. 3]

[FIG. 4]

<div style="text-align:center">

**INTERNATIONAL SEARCH REPORT**

</div>

| International application No. |
| --- |
| PCT/KR2020/002809 |

**A. CLASSIFICATION OF SUBJECT MATTER**

*C07K 14/435(2006.01)i, C07K 14/765(2006.01)i, A61K 9/00(2006.01)i, A61K 38/17(2006.01)i, A61K 47/64(2017.01)i, A61P 21/00(2006.01)i*

According to International Patent Classification (IPC) or to both national classification and IPC

**B. FIELDS SEARCHED**

Minimum documentation searched (classification system followed by classification symbols)

C07K 14/435; A23K 20/147; A23K 20/153; A61K 47/64; C07K 14/765; A61K 9/00; A61K 38/17; A61P 21/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: albumin, Slit3, LRRD2, muscle

**C. DOCUMENTS CONSIDERED TO BE RELEVANT**

| Category* | Citation of document, with indication, where appropriate, of the relevant passages | Relevant to claim No. |
| --- | --- | --- |
| X | KIM, B.-J. et al. Osteoclast-secreted SLIT3 coordinates bone resorption and formation. The Journal of clinical investigation. 2018, vol. 128, no. 4, pages 1429-1441 See abstract; page 1439. | 1-3 |
| Y | | 4-14 |
| Y | NCBI. GenBank Accession No. AAA98797.1. albumin [Homo sapiens]. 03 May 1996 See the entire document. | 4 |
| Y | KR 10-2017-0138920 A (THE ASAN FOUNDATION et al.) 18 December 2017 See abstract; sequence identifier nos. 1, 4; paragraph [0105]; claims 1-2, 9-10. | 4,11-13 |
| Y | KR 10-2019-0003746 A (JANSSEN BIOTECH, INC.) 09 January 2019 See abstract; claims 1, 5, 8-9, 15-21. | 5-10,14 |
| Y | NCBI. GenBank Accession No. AAQ89243.1. SLIT3 [Homo sapiens]. 03 October 2003 See the entire document. | 4 |

☐ Further documents are listed in the continuation of Box C. ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 16 JUNE 2020 (16.06.2020) | **16 JUNE 2020 (16.06.2020)** |

| Name and mailing address of the ISA/KR | Authorized officer |
| --- | --- |
| Korean Intellectual Property Office Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

| | International application No. |
|---|---|
| | **PCT/KR2020/002809** |

| Patent document cited in search report | Publication date | Patent family member | Publication date |
|---|---|---|---|
| KR 10-2017-0138920 A | 18/12/2017 | CN 109310740 A | 05/02/2019 |
| | | EP 3470078 A1 | 17/04/2019 |
| | | JP 2019-529334 A | 17/10/2019 |
| | | KR 10-2011957 B1 | 19/08/2019 |
| | | US 2019-0133173 A1 | 09/05/2019 |
| | | WO 2017-213435 A1 | 14/12/2017 |
| KR 10-2019-0003746 A | 09/01/2019 | AR 108442 A1 | 22/08/2018 |
| | | AU 2017-263237 A1 | 22/11/2018 |
| | | BR 112018072946 A2 | 19/02/2019 |
| | | CA 3022865 A1 | 16/11/2017 |
| | | CL 2018003148 A1 | 28/12/2018 |
| | | CN 109451726 A | 08/03/2019 |
| | | CO 2018012096 A2 | 22/11/2018 |
| | | CR 20180532 A | 04/03/2019 |
| | | EA 201892561 A1 | 30/04/2019 |
| | | EC SP18083561 A | 30/11/2018 |
| | | EP 3454832 A1 | 20/03/2019 |
| | | JP 2019-523637 A | 29/08/2019 |
| | | MX 2018013784 A | 28/03/2019 |
| | | NI 201800121 A | 18/02/2019 |
| | | PE 20190352 A1 | 07/03/2019 |
| | | SG 11201809700 A | 29/11/2018 |
| | | SV 2018005781 A | 28/03/2019 |
| | | TW 201803892 A | 01/02/2018 |
| | | US 10336812 B2 | 02/07/2019 |
| | | US 2017-0327560 A1 | 16/11/2017 |
| | | US 2019-0292241 A1 | 26/09/2019 |
| | | WO 2017-196647 A1 | 16/11/2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020170138920

### Non-patent literature cited in the description

- **H. NEURATH ; R.L.HILL.** The Proteins. Academic Press, 1979
- **MERRIFLELD.** *J. Amer. Chem. Soc.,* 1963, vol. 85, 2149-2156
- **SAMBROOK et al.** Molecular Cloning. Cold Spring Harbour Laboratory Press, 1989
- **SAMBROOK et al.** Molecular Cloning, A Laboratory Manual. Cold Spring Harbor Laboratory Press, 2001
- **YANOFSKY, C.** *J. Bacteriol.,* 1984, vol. 158, 1018-1024
- **HERSKOWITZ, I. ; HAGEN, D.** *Ann. Rev. Genet.,* 1980, vol. 14, 399-445
- *Appl. Environ. Microbiol.,* 1998, vol. 64, 3932-3938
- *Mol. Gen. Genet.,* 1996, vol. 250, 734-741
- **COHEN, S.N. et al.** *Proc. Natl. Acac. Sci. USA,* 1973, vol. 9, 2110-2114
- **HANAHAN, D.** *J. Mol. Biol.,* 1983, vol. 166, 557-580
- Remington's Pharmaceutical Science. Mack Publishing Company, 1975, 18042